(19) Europäisches Patentamt
European Patent Office
Office européen des brevets

(11) EP 0 841 337 B1

(12) **FASCICULE DE BREVET EUROPEEN**

(45) Date de publication et mention de la délivrance du brevet:
**05.09.2001 Bulletin 2001/36**

(21) Numéro de dépôt: **97402654.4**

(22) Date de dépôt: **06.11.1997**

(51) Int Cl.[7]: **C07D 491/056**, A61K 31/435, C07D 319/24, A61K 31/335, C07D 491/147
// (C07D491/056, 319:00, 221:00), (C07D491/056, 319:00, 209:00), (C07D491/147, 319:00, 221:00, 209:00)

(54) **Dérivés 7,12-dioxa-benzo [a] anthracéniques substitués, leur procédé de préparation et les compositions pharmaceutiques qui les contiennent**

Substituierte 7,12-Dioxabenzo(a)anthracenderivate, Verfahren zu ihrer Herstellung und diese enthaltende pharmazeutische Zubereitungen

Substituted 7,12-dioxabenzo(a)anthracene derivatives, process for their preparation and pharmaceutical compositions containing them

(84) Etats contractants désignés:
**AT BE CH DE DK ES FI FR GB GR IE IT LI LU NL PT SE**

(30) Priorité: **08.11.1996 FR 9613653**

(43) Date de publication de la demande:
**13.05.1998 Bulletin 1998/20**

(73) Titulaire: **ADIR ET COMPAGNIE**
**92415 Courbevoie Cédex (FR)**

(72) Inventeurs:
- **Coudert, Gérard**
  **45560 Saint Denis En Val (FR)**
- **Khatib, Siham, Résidence Aristote 2 app. 2424**
  **45100 Orleans (FR)**
- **Moreau, Pascale**
  **63000 Clermont Ferrand (FR)**
- **Caignard, Daniel-Henri**
  **78230 Le Pecq (FR)**
- **Renard, Pierre**
  **78000 Versailles (FR)**
- **Atassi, Ghanem**
  **92210 Saint Cloud (FR)**
- **Pierre, Alain**
  **78580 Les Alluets Le Roi (FR)**

(56) Documents cités:
**WO-A-90/08142**

- **M. NASR ET AL.: "Synthesis of benzoxazino- and naphtoxazinoquinoline derivatives as possible antitumor agents" JOURNAL OF PHARMACEUTICAL SCIENCES., vol. 63, no. 8, 1974, WASHINGTON US, pages 1314-1316, XP002035733**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 841 337 B1

Printed by Jouve, 75001 PARIS (FR)

## Description

La présente invention concerne de nouveaux dérivés 7,12-dioxa-benzo[a]anthracéniques substitués, leur procédé de préparation et les compositions pharmaceutiques qui les contiennent. Les composés de la présente invention trouvent une utilisation thérapeutique intéressante grâce à leur activité antitumorale.

Dans la littérature, le composé 7,12-dioxa-9,10-dichloro-benzo[a]anthracène (Biochem. Pharmacol., 1990, 40 (4), pp. 737-741), intéragissant avec le récepteur Ah (hydrocarbure Aromatique) du placenta humain, est décrit. Toutefois, aucune activité thérapeutique n'a été rapportée pour ce dérivé. De même, le composé 7,12-dioxa-benzo[a]anthracène est décrit dans Cancer Lett., 1987, 34(2), pp. 129-137. Enfin, le document WO 90/08142 revendique des dérivés substitués de dibenzo-p-dioxins et décrit leur propriété antiestrogénique et le J. Pharm. Sciences, 1974, 63 (8), pp. 1314-1316 décrit la synthèse de dérivés benzoxazinoquinoline qui possèdent des propriétés antitumorales.

Les composés de la présente invention trouvent leur originalité à la fois dans leur structure et dans leur utilisation en tant qu'agents antitumoraux.

Plus spécifiquement, la présente invention concerne les composés de formule (I) :

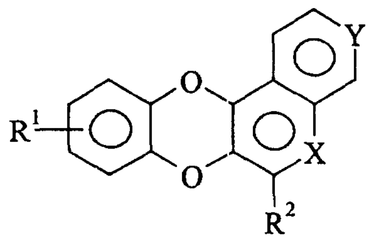

(I)

dans laquelle :

$R^1$ représente un atome d'hydrogène ou un groupement de formule O-R, dans lequel R représente un atome d'hydrogène, un groupement alkyle ($C_1$-$C_6$) linéaire ou ramifié, (éventuellement substitué par un groupement aryle ou hétéroaryle)

$R^2$ représente un atome d'hydrogène, un atome d'halogène, un groupement hydroxy, alkoxy ($C_1$-$C_6$) linéaire ou ramifié, formyle, $CF_3SO_3$, cyano, alkoxycarbonyle ($C_1$-$C_6$) linéaire ou ramifié, aryloxycarbonyle, NR'aR'b dans lequel R'a représente un groupement dialkylaminoalkyle ($C_1$-$C_6$) (chaque partie alkyle étant constituée par une chaîne comportant de 1 à 6 atomes de carbone, linéaire ou ramifiée, identique ou différente indépendamment l'une de l'autre), et R'b représente un atome d'hydrogène ou un groupement alkyle ($C_1$-$C_6$) linéaire ou ramifié, ou un groupement BNR"aR"b dans lequel B représente un groupement carbonyle ou méthylène, R"a et R"b ont respectivement la même définition que R'a et R'b ou R"a représente un groupement alkyle ($C_1$-$C_6$) linéaire ou ramifié substitué par au moins un groupement hydroxy,

X représente un atome d'azote (éventuellement substitué par un groupement R'c alkyle ($C_1$-$C_6$) linéaire ou ramifié), ou un groupement C-$R^3$ dans lequel $R^3$ représente un atome d'hydrogène, un groupement alkoxycarbonyle ($C_1$-$C_6$) linéaire ou ramifié, ou un groupement BNR"aR"b dans lequel B représente un carbonyle ou un groupement méthylène et R"a et R"b ont la même définition que précédemment, ou $R^2$ et X, lorsque X représente un groupement C-$R^3$, avec l'atome de carbone qui les porte forment ensemble un cycle de formule (II) :

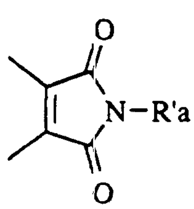

(II)

dans laquelle R'a a la même définition que précédemment,

Y représente un atome d'azote ou un groupement C-$R^4$ dans lequel $R^4$ représente un atome d'hydrogène, ou un groupement de formule O-R" dans laquelle R" représente un atome d'hydrogène, un groupement alkyle ($C_1$-$C_6$)

linéaire ou ramifié (éventuellement substitué par un groupement hydroxy), un groupement aryle ou hétéroaryle,

étant entendu que lorsque X représente un atome d'azote et Y un groupement CH, ou lorsque X représente un groupement CH et Y un atome d'azote ou lorsque X et Y représentent simultanément un groupement CH alors $R^1$ et $R^2$ ne peuvent représenter simultanément un atome d'hydrogène,
étant entendu que le terme aryle désigne un groupement phényle ou naphtyle éventuellement substitué de façon identique ou différente par un ou plusieurs atomes d'halogène ou groupements alkyle ($C_1$-$C_6$) linéaire ou ramifié, hydroxy, alkoxy ($C_1$-$C_6$) linéaire ou ramifié ou trihalogénométhyle,
et le terme hétéroaryle désigne un groupement aromatique mono ou bicyclique contenant 1 ou 2 hétéroatomes choisis parmi O, S, N éventuellement substitué de façon identique ou différente par un ou plusieurs atomes d'halogène ou groupements alkyle ($C_1$-$C_6$) linéaire ou ramifié, hydroxy, alkoxy ($C_1$-$C_6$) linéaire ou ramifié ou trihalogénométhyle,
leurs isomères ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Plus particulièrement, la présente invention concerne:

- les composés de formule (I) dans laquelle $R^2$ représente un groupement NR'aR'b dans lequel R'a représente un groupement dialkyle ($C_1$-$C_6$) -linéaire ou ramifié- aminoalkyle ($C_1$-$C_6$) linéaire ou ramifié, chaque partie alkyle étant identique ou différente indépendamment l'une de l'autre, et R'b représente un atome d'hydrogène ou un groupement alkyle ($C_1$-$C_6$) linéaire ou ramifié,
- ou les composés de formule (I) dans laquelle $R^2$ ou X, lorsque X consiste en un groupement C-$R^3$, représente un groupement BNR"aR"b dans lequel B est tel que défini précédemment, et R"a et R"b correspondant respectivement à R'a et R'b sont tels que définis précédemment,
- ou les composés de formule (I) tels que $R^2$ et X, lorsque X représente un groupement C-$R^3$, forment ensemble avec l'atome de carbone qui les porte un cycle de formule (II) :

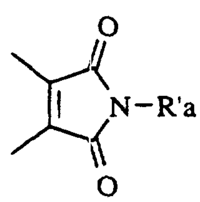

(II)

dans laquelle R'a a la même définition que précédemment, et plus particulièrement les composés de formule (I) tels que $R^2$ et X, lorsque X représente un groupement C-$R^3$ forment ensemble avec l'atome de carbone qui les porte un cycle de formule (II) dans laquelle R'a représente un groupement diméthylaminoéthyle.

La présente invention concerne plus spécifiquement le composé de formule (I) qui est le N2-(7,12-dioxa-3-hydroxy-benzo[a]anthracène-5,6-maléimido)-N1,N1-diméthyléthylènediamine, chlorhydrate.

L'invention s'étend également au procédé de préparation des composés de formule (I) caractérisé en ce que l'on utilise comme produit de départ

- *pour les composés où X représente un atome d'azote ou un groupement CH*, le composé de formule (III) :

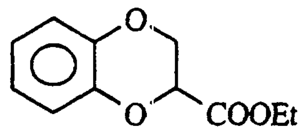

(III)

qui est - soit dibromé puis traité à l'acide métachloroperbenzoïque et soumis à l'action du l'iodure de potassium pour obtenir le composé (IIIa) :

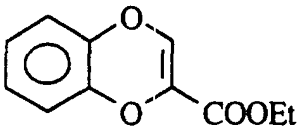

(IIIa)

- soit acylé selon une réaction de Friedel Crafts, dibromé puis traité à l'acide métachloroperbenzoïque, pour conduire au composé de formule (IV) :

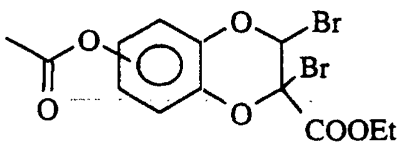

(IV)

qui, soumis à l'action de l'iodure de sodium, puis d'une base telle que l'éthylate de sodium, permet d'obtenir le composé (V) :

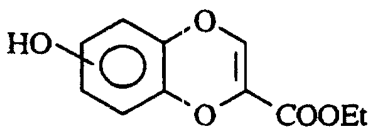

(V)

sur lequel on condense les électrophiles de formule R'''-Halogène, dans laquelle R''' représente un groupement alkyle ($C_1$-$C_6$) linéaire ou ramifié, éventuellement substitué par un groupement aryle ou hétéroaryle, pour conduire au composé de formule (VI) :

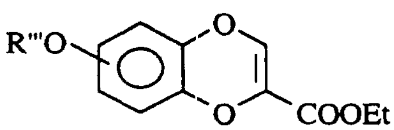

(VI)

dans laquelle R''' a la même définition que précédemment,
l'ensemble des composés (IIIa) et (VI) formant le composé de formule (VII) :

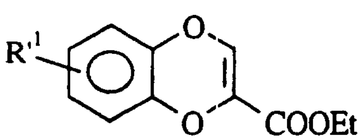

(VII)

dans laquelle $R'^1$ représente un atome d'hydrogène, un groupement alkoxy ($C_1$-$C_6$) linéaire ou ramifié, éventuellement substitué par un groupement aryle ou hétéroaryle,
sur lequel on condense, après saponification par l'hydroxyde de sodium aqueux par exemple, la diéthylamine en présence d'un agent de couplage comme le 1,3-diméthylaminopropyl-3-éthylcarbodiimide, pour conduire au composé de formule (VIII) :

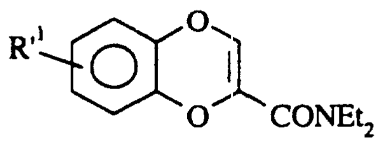

(VIII)

dans laquelle R'1 a la même signification que précédemment, qui est soumis à l'action d'une base forte, puis au chlorure de triméthylétain, pour conduire au composé de formule (IX):

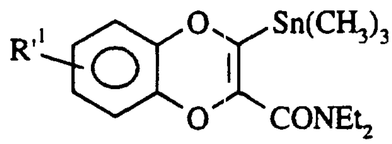

(IX)

dans laquelle R'[1] a la même signification que précédemment, sur lequel on condense, en présence d'iodure de cuivre et d'un complexe de métal de transition comme le palladium :

- **soit** la N-tert-butoxycarbonyl-2-iodoaniline, pour obtenir le composé de formule (X) :

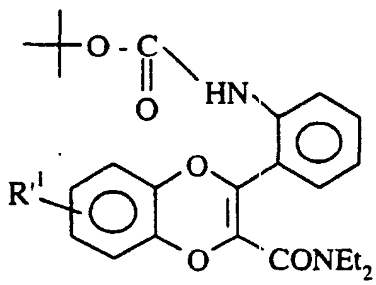

(X)

dans laquelle R'[1] a la même définition que précédemment, qui conduit après hydrolyse acide au composé de formule (XI) :

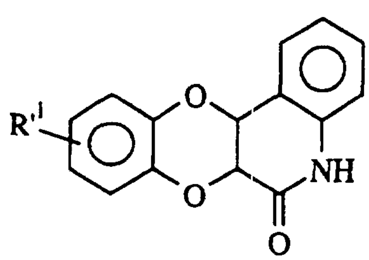

(XI)

dans laquelle R'[1] a la même définition que précédemment, sur lequel on fait agir $POCl_3$, pour obtenir le composé de formule (Va), cas particulier des composés de formule (I):

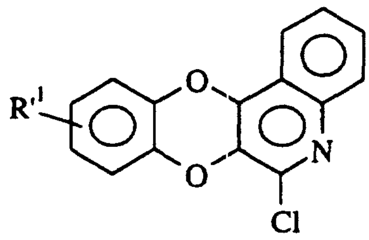

(I/a)

dans laquelle R'[1] est défini comme précédemment, que l'on soumet, dans le cas où R'[1] représente un groupement alkoxy ($C_1$-$C_6$) linéaire ou ramifié à un agent de déalkylation comme le tribromure de bore par exemple, pour conduire au composé de formule (I/b), cas particulier des composés de formule (I) :

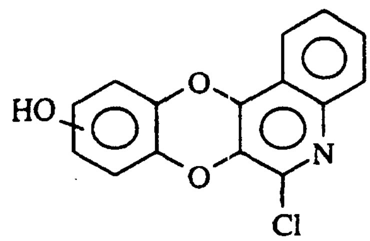

(I/b)

l'ensemble des composés (I/a) et (I/b) formant le composé de formule (I/c), cas particulier des composés de formule (I):

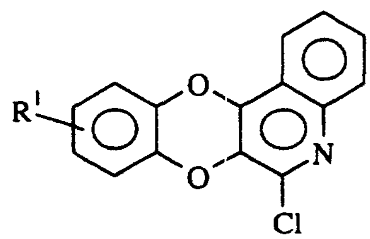

(I/c)

dans laquelle $R^1$ a la même signification que dans la formule (I), composé de formule (I/c) :

* que l'on soumet à un agent réducteur comme le zinc, pour conduire au composé de formule (I/d), cas particulier des composés de formule (I) :

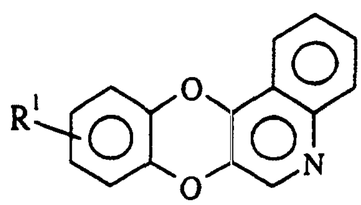

(I/d)

dans laquelle $R^1$ a la même signification que précédemment, sur lequel on effectue une N-alkylation par action d'un agent alkylant comme l'iodure de méthyle pour conduire au composé de formule (I/e), cas particulier des composés de formule (I):

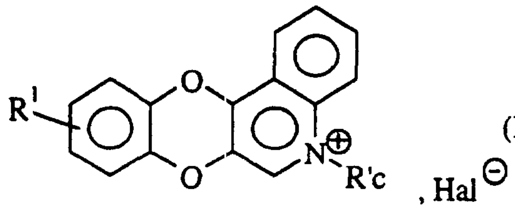

(I/e)

dans laquelle R'c représente un groupement alkyle ($C_1$-$C_6$) linéaire ou ramifié et Hal un halogène,

* ou, sur lequel on condense les diamines de formule HNR'aR'b où R'a représente un groupement dialkyle ($C_1$-$C_6$) -linéaire ou ramifié - aminoalkyle ($C_1$-$C_6$) linéaire ou ramifié, chaque partie alkyle étant identique ou différente indépendamment l'une de l'autre, et R'b représente un atome d'hydrogène ou un groupement alkyle ($C_1$-$C_6$) linéaire ou ramifié, pour conduire au composé de formule (I/f), cas particulier des composés de formule (I) :

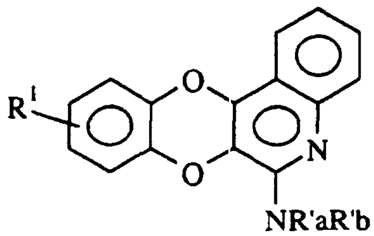

(I/f)

dans laquelle $R^1$, R'a et R'b ont la même signification que précédemment,

- **soit** le 2-iodotoluène pour obtenir le composé de formule (XII) :

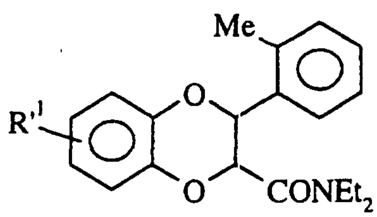

(XII)

dans laquelle $R'^1$ a la même signification que précédemment,
qui conduit, après l'action d'une base forte comme le diisopropylamidure de lithium au composé de formule (I/g), cas particulier des composés de formule (I):

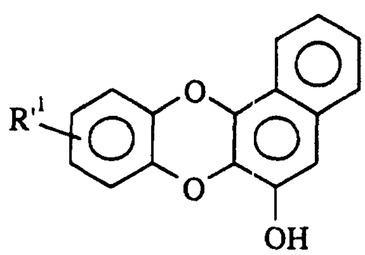

(I/g)

dans laquelle $R'^1$ est tel que défini précédemment,
composé de formule (I/g) que l'on soumet :

* dans le cas où $R'^1$ représente un groupement alkoxy ($C_1$-$C_6$)linéaire ou ramifié à un agent de déalkylation

comme le tribromure de bore par exemple, pour conduire au composé de formule (I/h), cas particulier des composés de formule (I):

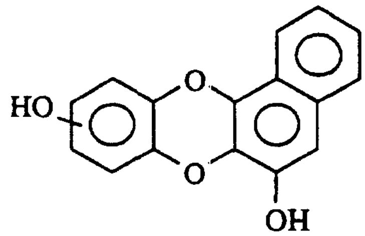

(I/h)

* ou à l'action successive de l'anhydride trifluorométhanesulfonique, d'un cyanure en présence d'un complexe de métal de transition comme le palladium, d'un agent réducteur comme l'hydrure de diisobutyl aluminium, pour obtenir le composé de formule (I/i), cas particulier des composés de formule (I):

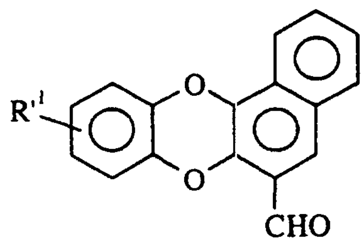

(I/i)

dans laquelle R'[1] a la même définition que précédemment, qui est oxydé en ester méthylique par l'action d'un agent oxydant comme l'oxyde de manganèse en présence de méthanol, pour conduire au composé de formule (I/j), cas particulier des composés de formule (I) ;

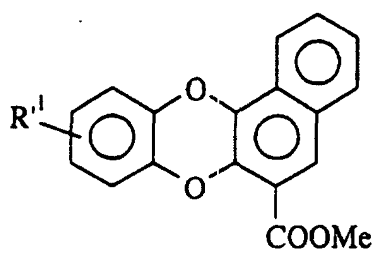

(I/j)

dans laquelle R'[1] a la même définition que précédemment, sur lequel on fait agir, dans le cas où R'[1] représente un groupement alkoxy ($C_1$-$C_6$)linéaire ou ramifié, un agent de déalkylation comme le tribromure de bore, pour conduire au composé de formule (I/k), cas particulier des composés de formule (I) :

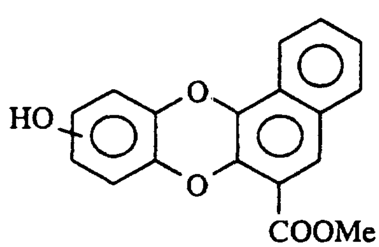

(I/k)

l'ensemble des composés (I/j) et (I/k) formant le composé de formule (I/l), cas particulier des composés de formule (I) :

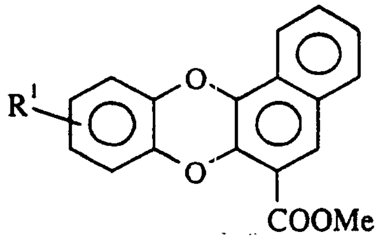

(I/l)

dans laquelle $R^1$ a la même définition que dans la formule (I), sur lequel on condense les diamines de formule HNR'aR'b où R'a représente un groupement dialkyle ($C_1$-$C_6$) -linéaire ou ramifié- aminoalkyle ($C_1$-$C_6$) linéaire ou ramifié, (chaque partie alkyle étant identique ou différente) et R'b représente un atome d'hydrogène ou un groupement alkyle ($C_1$-$C_6$) linéaire ou ramifié, pour conduire au composé de formule (I/m), cas particulier des composés de formule (I):

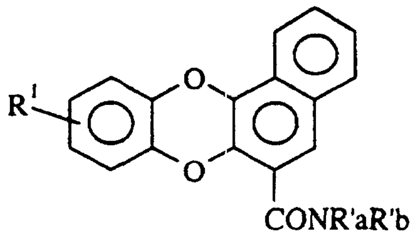

(I/m)

dans laquelle $R^1$, R'a et R'b ont la même signification que précédemment, étant entendu que les composés (I/m) peuvent être obtenus à partir du composé (I/k) soumis aux électrophiles de formule $R'^1$-Halogène où $R'^1$ est tel que défini précédemment, en milieu basique,

- *pour les composés où X ne représente pas un atome d'azote,* le composé de formule (XIII) :

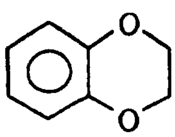

(XIII)

qui est :

- soit dibromé puis traité à l'acide métachloroperbenzoïque et soumis à l'action de l'iodure de potassium pour obtenir le composé (XIIIa) :

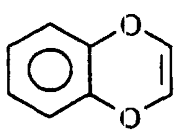

(XIIIa)

- soit acylé selon une réaction de Friedel Crafts, dibromé, traité à l'acide métachloroperbenzoique, soumis à l'action de l'iodure de sodium puis d'une base pour obtenir le composé (XIV) :

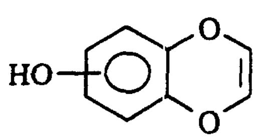

(XIV)

sur lequel on condense les électrophiles de formule R'''-Halogène dans laquelle R''' représente un groupement alkyle ($C_1$-$C_6$) linéaire ou ramifié, éventuellement substitué par un groupement aryle ou hétéroaryle, pour conduire au composé de formule (XV) :

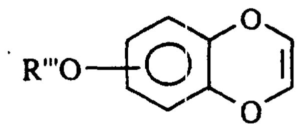

(XV)

dans laquelle R''' a la même définition que précédemment, l'ensemble des composés (XIIIa) et (XV) formant le composé de formule (XVI) :

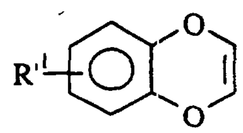

(XVI)

dans laquelle $R'^1$ représente un atome d'hydrogène, un groupement alkoxy ($C_1$-$C_6$) linéaire ou ramifié, éventuellement substitué par un groupement aryle ou hétéroaryle, sur lequel on condense, après l'action d'une base forte comme le butyl lithium par exemple, les électrophiles de formule (XVII):

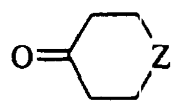

(XVII)

dans laquelle Z représente un groupement NCOOEt ou un groupement

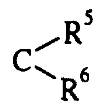

où $R^5$ et $R^6$ représentent simultanément un atome d'hydrogène ou forment ensemble un cycle

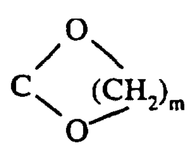

où m vaut 2 ou 3,
pour conduire au composé de formule (XVIII) :

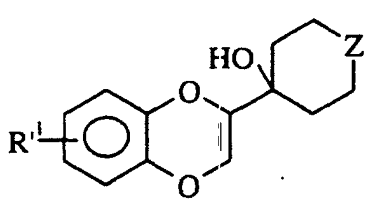

(XVIII)

dans laquelle $R'^1$ et Z ont la même définition que précédemment, qui est déshydraté en présence de chlorure de mésyle

par exemple, pour obtenir le composé de formule (XIX) :

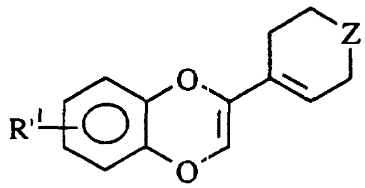

(XIX)

dans laquelle R'1 et Z sont tels que définis précédemment, sur lequel on condense l'acétylène dicarboxylate de méthyle ou le propiolate de méthyle pour obtenir le composé de formule (XX) :

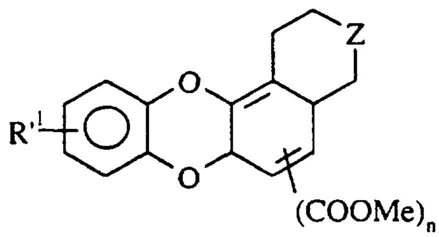

(XX)

dans laquelle R'1 et Z ont la même signification que précédemment et n vaut 1 ou 2, que l'on soumet à l'action d'un agent oxydant comme l'orthochloranile ou la 2,3-dichloro-5,6-dicyano-1,4-benzoquinone pour conduire

- au composé de formule (I/n), cas particulier des composés de formule (I) :

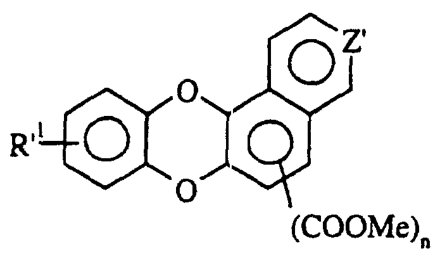

(I/n)

dans laquelle R'1 et n ont la même définition que précédemment et Z' représente un atome d'azote ou un groupement C-$R^7$ où $R^7$ représente un atome d'hydrogène ou un groupement O-$(CH_2)_m$-OH dans lequel m vaut 2 ou 3,
- ou au composé de formule (XXI) :

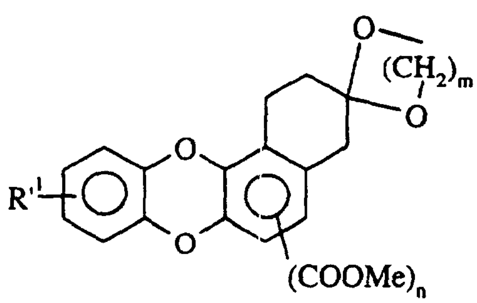

(XXI)

dans laquelle R'1, n et m sont tels que définis précédemment, qui est successivement soumis au déblocage de l'acétal en milieu acide, à la formation d'un éther silylé, à l'oxydation par la 2,3-dichloro-5,6-dicyano-1,4-benzoquinone, et déblocage de l'éther silylé par un agent fluoré, pour conduire au composé de formule (I/o), cas particulier des composés de formule (I) :

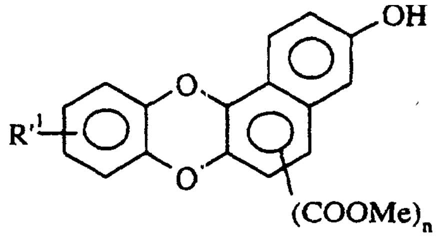

(I/o)

dans laquelle R'[1] et n ont la même définition que précédemment, sur lequel on condense divers électrophiles $R^8$-Halogène où $R^8$ représente un groupement alkyle ($C_1$-$C_6$) linéaire ou ramifié, éventuellement substitué par un groupement aryle ou hétéroaryle, pour obtenir les composés de formule (I/p), cas particulier des composés de formule (I) :

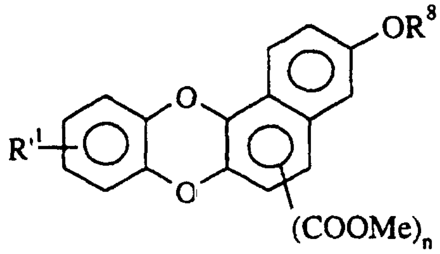

(I/p)

dans laquelle R'[1], n et $R^8$ sont tels que définis précédemment, les composés de formules (I/n), (I/o) et (I/p) formant l'ensemble des composés de formule (I/q), cas particulier des composés de formule (I) :

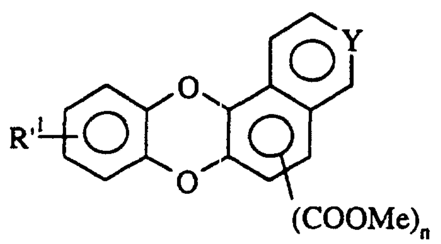

(I/q)

dans laquelle R'[1] et n sont tels que définis précédemment et Y a la même définition que dans la formule (I), composé (I/q) sur lequel :

* lorsque n vaut 1 ou 2, on condense les diamines de formule HNR"aR"b dans laquelle R"a représente un groupement dialkyle ($C_1$-$C_6$) -linéaire ou ramifié- aminoalkyle ($C_1$-$C_6$) linéaire ou ramifié (chaque partie alkyle étant identique ou différente indépendamment l'une de l'autre) ou un groupement alkyle ($C_1$-$C_6$) linéaire ou ramifié, substitué par au moins un groupement hydroxy, et R"b représente un atome d'hydrogène ou un groupement alkyle ($C_1$-$C_6$) linéaire ou ramifié pour conduire aux composés de formule (I/r), cas particulier des composés de formule (I) :

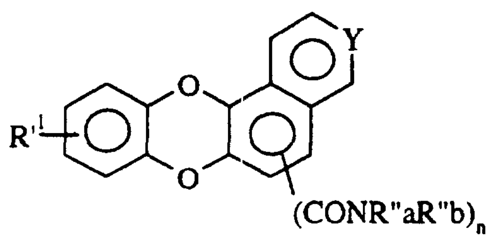

(I/r)

dans laquelle R'[1], R"a, R"b, n et Y ont la même définition que précédemment,

* ou, lorsque n vaut 1 ou 2, que l'on soumet à des conditions réductrices pour conduire à l'alcool correspondant qui subit une oxydation en aldéhyde en présence d'oxyde de manganèse pour conduire au composé de formule (XXII):

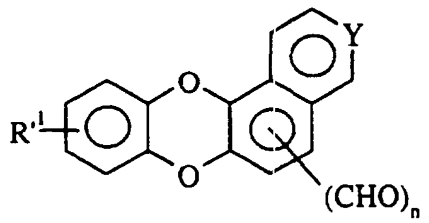

(XXII)

dans laquelle R'[1], Y et n sont tels que définis précédemment,
qui est condensé, en présence de chlorure de zinc et de cyanoborohydrure de sodium, avec les diamines de formule HNR"aR"b dans laquelle R"a représente un groupement dialkyle ($C_1$-$C_6$) -linéaire ou ramifié- aminoalkyle ($C_1$-$C_6$) linéaire ou ramifié ou un groupement alkyle ($C_1$-$C_6$) linéaire ou ramifié, substitué par au moins un groupement hydroxy, et R"b représente un atome d'hydrogène ou un groupement alkyle ($C_1$-$C_6$) linéaire ou ramifié pour conduire aux composés de formule (I/s), cas particulier des composés de formule (I):

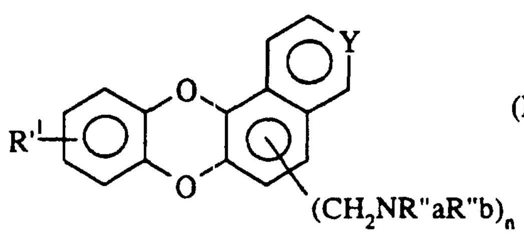

(I/s)

dans laquelle R'[1], R"a, R"b, Y et n ont la même définition que précédemment,

* ou lorsque n vaut 2 aux composés de formule (I/t), cas particulier des composés de formule (I):

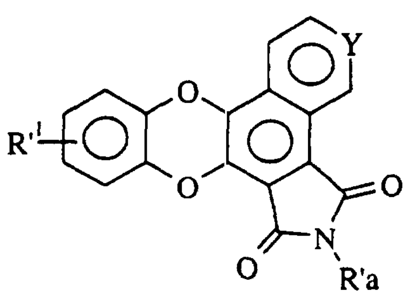

(I/t)

dans laquelle R'[1], R'a et Y ont la même définition que précédemment,

les composés (Va-t) formant l'ensemble des composés de l'invention que l'on purifie, le cas échéant, selon une technique classique de purification, qui peuvent, si on le désire, être séparés en leurs différents isomères optiques ou géométriques ou salifiés avec un acide ou une base pharmaceutiquement acceptable.

Les composés de départ utilisés dans le procédé précédemment décrit sont commerciaux ou aisément accessibles à l'homme du métier selon des procédés bien connus dans la littérature comme par exemple ceux décrits par J. Koo, S. Avakian, G.J. Martin, J. Am. Chem. Soc., 77, 1955, p. 5373.

Les composés de formule (I) possèdent d'intéressantes propriétés pharmacologiques. Ils ont une excellente cytotoxicité *in vitro* non seulement sur des lignées leucémiques mais également sur des lignées de tumeurs solides, ils ont également une action sur le cycle cellulaire et sont actifs *in vivo,* sur un modèle leucémique. Ces propriétés permettent leur utilisation en thérapeutique en tant qu'agents antitumoraux.

La présente invention a également pour objet les compositions pharmaceutiques contenant les produits de formule (I), leurs isomères optiques ou un de leurs sels d'addition à une base ou un acide pharmaceutiquement acceptables, seul ou en combinaison avec un ou plusieurs excipients ou véhicules inertes, non toxiques.

Parmi les compositions pharmaceutiques selon l'invention, il sera cité plus particulièrement celles qui conviennent pour l'administration orale, parentérale, nasale, rectale, perlinguale, oculaire ou respiratoire, et notamment les comprimés simples ou dragéifiés, les comprimés sublinguaux, les sachets, les paquets, les gélules, les glossettes, les tablettes, les suppositoires, les crèmes, les pommades, les gels dermiques, les préparations injectables ou buvables, les aérosols, les gouttes oculaires ou nasales.

La posologie utile varie selon l'âge et le poids du patient, la voie d'administration, la nature de l'indication thérapeutique et des traitements éventuellement associés et s'échelonne entre 0,1 et 400 mg par jour, en une ou plusieurs administrations.

Les exemples suivants illustrent l'invention et ne la limitent en aucune façon. Les produits de départ sont connus ou préparés à partir de modes opératoires connus.

**EXEMPLE 1 : 7,12-dioxa-5-aza-benzo[a]anthracène**

*Stade A : Acide benzo[1,4]dioxine-2-carboxylique*

1 mmole de benzo[1,4]dioxine-2-carboxylate d'éthyle est portée à reflux dans 25 ml d'une solution d'hydroxyde de sodium à 8 %. Après refroidissement, une solution d'acide chlorhydrique 3N est ajoutée lentement jusqu'à précipitation de l'acide benzodioxinique. Après filtration, le solide est recristallisé dans du benzène.
*Point de fusion : 178-180° C*

*Stade B : Benzo[1,4]-dioxine-2-diéthyl-carboxamide*

Sous atmosphère inerte, 15,4 mmoles de diéthylamine, 15,4 mmoles de 1,3-diméthylaminopropyl-3-éthylcarbodiimide et 15,4 mmoles d'hydroxy-benzotriazole sont ajoutées à une solution de 14,04 mmoles d'acide benzo[1,4] dioxine-2-carboxylique dans 25 ml de DMF à 0° C. Le mélange est ramené à température ambiante et laissé agité 18 heures. Après évaporation de la DMF, le résidu est lavé à l'eau puis extrait à l'acétate d'éthyle. La phase organique est ensuite séchée sur sulfate de magnésium et concentrée sous vide. L'amide est purifié par chromatographie sur gel de silice (éluant : éther de pétrole/acétate d'éthyle).

*Stade C : 3-Triméthylstannyl-benzo[1,4]dioxine-2-diéthylcarboxamide*

A -78° C, sous atmosphère inerte, 17,4 mmoles de diisopropylamidure de lithium sont ajoutées à 8,58 mmoles de benzo[1,4]dioxine-2-diéthylcarboxamide en solution dans 50 ml de tétrahydrofurane anhydre. Après 2 h 30 min de réaction, 21,4 mmoles de chlorure de triméthylétain dissous dans un minimum de tétrahydrofurane sont additionnées goutte à goutte. Le mélange réactionnel est hydrolysé après 3 heures puis extrait à l'acétate d'éthyle. La phase organique est séchée sur sulfate de magnésium et concentrée sous vide. Le dérivé stannique est purifié par chromatographie éclair sur gel de silice traité à la triéthylamine.
*Point de fusion : 63-65° C*

*Stade D : 3-[2-(t-Butyloxycarbonyl-amino)phényl]-benzo[1,4]dioxine-2-diéthylcarboxamide*

Sous atmosphère inerte, 3,93 mmoles de 3-triméthylstannyl-benzo[1,4]dioxine-2-diéthylcarboxamide et 3,94 mmoles de N-t-butyloxycarbonyl-2-iodoaniline sont dissoutes dans 25 ml de 1,4-dioxane. Le mélange réactionnel est porté à reflux après addition de 5 % de palladium tetrakis (triphénylphosphine) et 5 % d'iodure cuivreux. Le solvant est évaporé sous vide, le résidu est extrait à l'acétate d'éthyle. La phase organique est séchée sur sulfate de magnésium puis concentrée sous vide. L'amide est purifiée par chromatographie sur gel de silice (éluant : éther de pétrole/acétate d'éthyle).
*Point de fusion : 147-149° C*

*Stade E : [5-H]-7,12-Dioxa-5-aza-benzo[a]anthracèn-6-one*

A 1,2 mmoles de 3-[2-(t-butyloxycarbonyl-amino)phényl]-benzo[1,4]dioxine-2-diéthylcarboxamide dans 25 ml de 1,4-dioxane sont ajoutés 25 ml d'acide chlorhydrique 6N. Le mélange est chauffé à 70° C pendant 8 heures. Le volume de dioxane est réduit de moitié sous vide avant de filtrer le mélange. Le solide est lavé à l'eau puis séché sous vide en présence de pentoxyde de phosphore.
*Point de fusion : > 300° C*

*Stade F : 6-Chloro-5-aza-7,12-dioxa-benzo[a]anthracène*

Sous atmosphère inerte, la [5H]-7,12-dioxa-5-aza-benzo[a]anthracèn-6-one est portée à reflux d'oxychlorure de phosphore pendant 6 heures. Le solvant (oxychlorure de phosphore) est ensuite évaporé sous vide et le mélange brut est chromatographié par chromatographie éclair sur gel de silice (éluant : éther pétrole/acétate d'éthyle).
*Point de fusion : 136-138° C*

*Stade G : 7,12-dioxa-5-aza -benzo[a]anthracène*

Sous atmosphère inerte, 1 mmole de 6-chloro-5-aza-7,12-dioxa-benzo[a]anthracène et 8 mmoles de zinc en poudre sont portées à 50°C dans 5 ml d'acide acétique pendant 8 heures. Après refroidissement, le mélange réactionnel est dilué dans du méthanol puis filtré sur célite. Le résidu est concentré sous vide puis lavé avec une solution d'hydrogénocarbonate de sodium saturée puis extrait avec du chloroforme. La phase organique est séchée sur sulfate de magnésium et concentrée sous vide. Le mélange brut est - chromatographié par chromatographie éclair sur gel de silice (éluant : acétate d'éthyle/éther de pétrole).
*Point de fusion : 127-129°* C

**EXEMPLE 2 : N2-(7,12-dioxa-5-aza-benzo[a]anthracèn-6-yl)-N1,N1-diméthyléthane-1,2-diamine**

Sous atmosphère inerte, le 6-chloro-5-aza-7,12-dioxa-benzo[a]anthracène (composé de l'exemple 1 - Stade F) est porté à reflux de N,N-diméthyléthane-1,2-diamine pendant 2 heures. La diamine en excès est évaporée sous vide et le brut résiduel est chromatographié par chromatographie éclair sur gel de silice traité à la triéthylamine (éluant : acétate d'éthyle/méthanol).

**EXEMPLE 3 : N2-(7,12-dioxa-5-aza-benzo[a]anthracèn-6-yl)-N1,N1-diméthyléthane-1,2-diamine, chlorhydrate**

Une solution d'éther saturée en vapeur d'acide chlorhydrique est additionnée à la N2-(7,12-dioxa-5-aza-benzo [a]anthracèn-6-yl)-N1,N1-diméthyléthane-1,2-diamine (composé de l'exemple 2) dissoute dans le minimum de dichlorométhane. Le chlorhydrate correspondant obtenu quantitativement est filtré, lavé à l'éther puis séché sous vide.
*Point de fusion :* 280-282° C

**EXEMPLE 4 : N2-(7,12-dioxa-5-aza-benzo[a]anthracèn-6-yl)-N1,N1-diéthyléthane-1,2-diamine, chlorhydrate**

La diamine est obtenu selon le procédé de l'exemple 2 avec la N,N-diéthyléthane-1,2-diamine et un temps réactionnel de 3 heures et le chlorhydrate est obtenu selon le procédé de l'exemple 3.
*Point de fusion : 256-258° C*

**EXEMPLE 5 : N2-(7,12-dioxa-5-aza-benzo[a]anthracèn-6-yl)-N1,N1-diméthylpropane-1,2-diamine, chlorhydrate**

La diamine est obtenue selon le procédé de l'exemple 2 avec la N,N-diméthyl-propane-1,2-diamine et le chlorhydrate selon l'exemple 3.
*Point de fusion : 238-240° C*

**EXEMPLE 6 : N2-(7,12-dioxa-5-aza-benzo[a]anthracèn-6-yl)-N1,N1-diéthylpropane-1,2-diamine, chlorhydrate**

La diamine est obtenue selon le procédé de l'exemple 2 avec la N,N-diéthyl-propane-1,2-diamine avec un temps réactionnel de 3 heures et le chlorhydrate selon le procédé de l'exemple 3.
*Point de fusion : 220-222° C*

**EXEMPLE 7: 10-Méthoxy-5-aza-7,12-dioxa-benzo[a]anthracène**

*Stade A : 6-Acétyl-benzo[1,4]dioxine-2-carboxylate d'éthyle*

12,5 g de trichlorure d'aluminium sont additionnés à un mélange de 4,27 g de chlorure d'acétyle et 7,5 g de benzo[1,4]dioxine-2-carboxylate d'éthyle dans 150 ml de sulfure de carbone à 0° C. Le mélange réactionnel est agité à température ambiante pendant 14 heures. Après hydrolyse acide avec 50 ml d'acide chlorhydrique (2N) et extraction de la phase aqueuse par le dichlorométhane, la phase organique est lavée par une solution saturée d'hydrogénocarbonate de sodium puis séchée sur sulfate de magnésium. Le produit acylé est purifié par chromatographie sur gel de

silice (éluant : éther de pétrole/acétate d'éthyle : 7/3).
*Point de fusion : 123-125° C*

*Stade B : 6-Acétyl-2,3-dibromo-benzo[1,4]dioxane-2-carboxylate d'éthyle*

Sous atmosphère inerte, à 0°C, 1,1 ml de brome élémentaire en solution dans 5 ml de tétrachlorure de carbone sont ajoutés goutte à goutte pendant 30 minutes à 5 g de 6-acétylbenzo[1,4]dioxine-2-carboxylate d'éthyle partiellement solubilisés dans 50 ml de tétrachlorure de carbone. Après 4 heures, le milieu réactionnel est concentré sous vide. Le dérivé dibromé est purifié par chromatographie éclair sur gel de silice (éluant : éther de pétrole/acétate d'éthyle : 8/2).
*Huile*

*Stade C : 6-Acétoxy-2,3-dibromo-benzo[1,4]dioxane-2-carboxylate d'éthyle*

Sous atmosphère inerte, 4 g de 6-acétyl-2,3-dibromo-benzo[1,4]dioxane-2-carboxylate d'éthyle sont portés à reflux de dichlorométhane en présence de 5,6 g d'acide métachloroperbenzoïque pendant 72 heures. L'acide chlorobenzoïque formé est éliminé par filtration et le filtrat est lavé par une solution saturée d'hydrogénocarbonate de potassium et une solution saturée de chlorure de sodium. La phase organique est séchée sur sulfate de magnésium et concentrée sous vide. Le mélange brut est chromatographié par chromatographie éclair sur gel de silice (éluant : éther de pétrole/acétate d'éthyle : 7/3).
*Point de fusion : 58-60° C*

*Stade D : 6-Acétoxy-benzo[1,4]dioxine-2-carboxylate d'éthyle*

Sous atmosphère inerte, 6 g de 6-acétoxy-2,3-dibromo-benzo[1,4]dioxane-2-carboxylate d'éthyle en solution dans 100 ml d'acétone sont mélangés à 7,75 g d'iodure de potassium et agités pendant 6 heures. Après évaporation de l'acétone sous pression réduite, le résidu est dilué dans l'éther puis lavé par une solution de thiosulfate de sodium à 20% jusqu'à décoloration du mélange. La phase organique est ensuite séchée sur sulfate de magnésium puis concentrée sous vide. Le composé est purifié par chromatographie sur gel de silice (éluant : éther de pétrole/acétate d'éthyle : 7/3).
*Point de fusion : 86-88° C*

*Stade E : 6-Hydroxy-benzo[1,4]dioxine-2-carboxylate d'éthyle*

Sous atmosphère inerte, 5 g de 6-acétoxy-benzo[1,4]dioxine-2-carboxylate d'éthyle en solution dans 75 ml d'éthanol et 3 ml d'éthanolate de sodium (1M) sont agités pendant 18 heures. Le milieu réactionnel est ensuite neutralisé par Dowex (X-8 forme acide) puis filtré et concentré sous vide. Le brut résiduel est purifié par chromatographie sur gel de silice (éluant : éther de pétrole/acétate d'éthyle : 7/3).
*Point de fusion : 179-181° C*

*Stade F : 6-Méthoxy-benzo[1,4]dioxine-2-carboxylate d'éthyle*

Sous atmosphère inerte, 2,4 g de 6-hydroxy-benzo[1,4]dioxine-2-carboxylate d'éthyle et 540 mg d'hydrure de sodium dans 25 ml de diméthylformamide sont agités à 0° C. Après 40 minutes, 0,84 ml de iodométhane sont additionnés goutte à goutte puis le mélange est agité 1 heure à température ambiante. Le solvant est évaporé sous vide, le résidu est dilué dans de l'acétate d'éthyle puis lavé à l'eau. La phase organique est séchée sur sulfate de magnésium puis concentrée sous vide. L'éther méthylique est purifié par chromatographie sur gel de silice (éluant : éther de pétrole/acétate d'éthyle : 7/3).
*Point de fusion : 98-100° C*
Le composé de l'exemple 7 est obtenu en reprenant les stades A, B, C, D, E, F et G de l'exemple 1 à partir du 6-méthoxy-benzo[1,4]dioxine-2-carboxylate d'éthyle.
*Point de fusion : 132-134° C*

**EXEMPLE 8 : 10-Benzyloxy-5-aza-7,12-dioxa-benzo[a]anthracène**

On utilise la même procédure que pour l'exemple 7 en remplaçant dans le stade F l'iodure de méthyle par du chlorure de benzyle.

**<u>EXEMPLE 9</u> : N2-(10-méthoxy-7,12-dioxa-5-aza-benzo[a]anthracèn-6-yl)-N1,N1-diméthyléthane-1,2-diamine**

Le composé de l'exemple 9 est obtenu en reprenant les stades A, B, C, D, E et F de l'exemple 7, puis les stades A, B, C, D, E et F de l'exemple 1 à partir du 6-méthoxy-benzo[1,4]dioxine-2-carboxylate d'éthyle et le stade H (exemple 2).
*<u>Point de fusion</u> : 148-150° C*

**<u>EXEMPLE 10</u> : N2-(10-hydroxy-7,12-dioxa-5-aza-benzo-[a]anthracèn-6-yl)-N1,N1-diméthyléthane-1,2-diamine, chlorhydrate**

*<u>Stade A</u> : 10-Méthoxy-6-chloro-5-aza-7,12-dioxa-benzo[a]anthracène*

On procède comme pour les stades A, B, C, D, E et F de l'exemple 7 puis A, B, C, D, E et F de l'exemple 1 en prenant comme substrat de départ le 6-méthoxy-benzo[1,4]dioxine-2-carboxylate d'éthyle.
*<u>Point de fusion</u> : 194-196° C*

*<u>Stade B</u> : 10-Hydroxy-6-chloro-7,12-dioxa-5-aza-benzo[a]anthracène*

Sous atmosphère inerte et à 0°C, 1,22 ml de tribromure de bore sont ajoutés goutte à goutte à 80 mg de 10-méthoxy-7,12-dioxa-6-chloro-5-aza-benzo[a]anthracène en solution dans 3 ml de dichlorométhane. Après 2 heures, le mélange réactionnel est hydrolysé par une solution saturée de carbonate de sodium. La phase organique est séchée sur sulfate de magnésium et concentrée sous vide. Le composé déméthylé est purifié par chromatographie sur gel de silice (éluant : éther de pétrole/acétate d'éthyle : 7/3).
*<u>Point de fusion</u> : > 300° C*

*<u>Stade C</u> : N2-(10-hydroxy-7,12-dioxa-5-aza-benzo[a]anthracèn-6-yl)-N1,N1-diméthyléthane-1,2-diamine, chlorhydrate*

On procède comme pour l'exemple 2 pour obtenir la diamine à partir du 10-hydroxy-6-chloro-7,12-dioxa-5-aza-benzo[a]anthracène et le chlorhydrate correspondant est obtenu en procédant comme dans l'exemple 3.
*<u>Point de fusion :</u> 273-275° C*

**<u>EXEMPLE 11</u> : 6-Hydroxy-10-méthoxy-7,12-dioxa-benzo[a]anthracène**

*<u>Stade A</u> : 3-Triméthylstannyl-6-méthoxy-benzo[1,4]dioxine-2-diéthyl carboxamide*

On procède comme pour les stades A, B, C, D, E et F de l'exemple 7 puis les stades A, B et C de l'exemple 1 à partir du 6-méthoxy-benzo[1,4]dioxine-2-carboxylate d'éthyle.
*<u>Point de fusion</u> ; 58-60° C*

*<u>Stade B</u> : 3-(2-Tolyl)-6-méthoxy-benzo[1,4]dioxine-2-diéthyl carboxamide*

Sous atmosphère inerte, 500 mg de 3-triméthylstannyl-6-méthoxy-benzo[1,4]dioxine-2-diéthyl carboxamide et 0,2 ml de iodotoluène sont portés à reflux de 1,4-dioxane en présence de 122 mg de palladium tétrakis et 22 mg d'iodure cuivreux pendant 3 heures. Le dioxane est ensuite évaporé sous vide et le résidu est lavé à l'eau puis extrait à l'acétate d'éthyle. La phase organique est séchée sur sulfate de magnésium et concentrée sous vide. Le produit de couplage est purifié par chromatographie éclair sur gel de silice (éluant : toluène/acétate d'éthyle : 95/5).
*<u>Point de usion</u> : 60-62° C*

*<u>Stade C</u> : 6-Hydroxy-10-méthoxy-7,12-dioxa-benzo[a]anthracène*

Sous atmosphère inerte, 1,42 ml de diisopropylamidure de lithium sont additionnés goutte à goutte à 0,95 mmoles de 3-(2-tolyl)-6-méthoxy-benzo[1,4]dioxine-2-diéthylcarboxamide en solution dans 7 ml de THF à -78° C. Le mélange réactionnel est maintenu à cette température pendant 2 à 3 heures. La température est ramenée à -10°C avant l'hydrolyse acide (HCl 10 %). La phase aqueuse est extraite au dichlorométhane puis la phase organique est séchée sur sulfate de magnésium et concentrée sous vide. Le produit cyclisé est purifié par chromatographie éclair sur gel de silice (éluant : toluène/acétate d'éthyle 95/5).
*<u>Point de fusion</u> : 183-185° C*

| *Microanalyse élémentaire :* | | | |
|---|---|---|---|
| | *C%* | *H%* | *O%* |
| *calculé* | *72,85* | *4,32* | *22,83* |
| *trouvé* | *72,57* | *4,61* | *22,86* |

**EXEMPLE 12 : 6,10-Dihydroxy-7,12-dioxa-benzo[a]anthracène**

Sous atmosphère inerte, 0,1 ml de tribromure de bore sont additionnés goutte à goutte à 90 mg de 6-hydroxy-10-méthoxy-7,12-dioxa-benzo[a]anthracène en solution dans 3 ml de dichlorométhane à 0° C. Après 4 heures, le mélange est hydrolysé. La phase organique est séchée sur sulfate de magnésium et concentrée sous vide. Le composé bis hydroxylé est purifié par chromatographie éclair sur gel de silice (éluant : éther de pétrole/acétate d'éthyle).
*Point de fusion : 219-221° C*

**EXEMPLE 13 : 10-Hydroxy-7,12-dioxa-benzo[a]anthracèn-6-(2-diméthylaminoéthyl) carboxamide, chlorhydrate**

*Stade A : 6-Isopropyloxybenzo[1,4]-dioxine-2-carboxylate d'éthyle*

On procède de la même façon que pour les stades A, B, C, D, E et F de l'exemple 7 avec l'iodure d'isopropyle au lieu du iodure de méthyle.

*Stade B : 3 -Triméthylstannyl-6-isopropyloxy-benzo[1,4]-dioxine-2-diéthyl carboxamide*

On procède comme pour les stades A, B et C de l'exemple 1 à partir du 6-isopropyloxybenzo[1,4]-dioxin-2-carboxylate d'éthyle.

*Stade C : 6-Hydroxy-10-isopropyloxy-7,12-dioxa -benzo[a]anthracène*

On procède comme dans les stades B et C de l'exemple 11.

*Stade D : 10-Isopropyloxy-7,12-dioxa-benzo[a]anthracèn-6-trifluorométhanesulfonique ester*

Sous atmosphère inerte, 0,1 ml de pyridine puis 0,45 ml d'anhydride triflique sont ajoutés goutte à goutte à 0°C au 6-hydroxy-10-isopropyloxy-7,12-dioxa-benzo[a]anthracène en solution dans 10 ml de dichlorométhane, Après 1 heure, le mélange réactionnel est hydrolysé avec une solution de chlorure d'ammonium saturée. On procède à une extraction avec du dichlorométhane, puis la phase organique est séchée sur sulfate de magnésium et concentrée sous vide. La purification se fait par chromatographie éclair sur gel de silice (éluant : éther de pétrole/acétate d'éthyle : 9/1).
*Point de fusion : 98-100° C*

*Stade E : 6-Cyano-10-isopropyloxy-7,12-dioxa-benzo[a]anthracène*

Sous atmosphère inerte, 27 mg de cyanure de zinc et 420 mg de palladium tétrakis sont additionnés successivement au dérivé triflique en solution dans 3 ml de DMF. Le mélange réctionnel est ensuite chauffé à 80°C pendant 30 minutes. Après évaporation de la DMF sous pression réduite, le résidu est lavé avec une solution de carbonate de sodium saturée puis extrait à l'acétate d'éthyle. La phase organique est séchée sur sulfate de magnésium et concentrée sous vide. On procède à une purification par chromatographie éclair sur gel de silice (éluant : éther de pétrole/acétate d'éthyle : 95/5).
*Point de fusion : 133-135°C*

*Stade F : 10-Isopropyloxy-7,12-dioxa-benzo[a]anthracèn-6-carbaldéhyde*

Sous atmosphère inerte, 0,8 ml d'hydrure de diisobutylaluminium sont additionnés goutte à goutte à 170 mg de 6-cyano-10-isopropyloxy-7,12-dioxa-benzo[a]anthracène en solution dans 5 ml de toluène à température ambiante. Après 15 minutes, le mélange réactionnel est hydrolysé avec une solution d'acide chlorhydrique à 5 %. La phase aqueuse est extraite au dichlorométhane, puis la phase organique est séchée sur sulfate de magnésium et concentrée

sous vide. L'aldéhyde est purifié par chromatographie éclair sur gel de silice (éluant : éther de pétrole/acétate d'éthyle : 95/5).
*Point de fusion : 140-142°C*

*Stade G : 10-Isopropyloxy-7,12-dioxa-benzo[a]anthracèn-6-carboxy méthylester*

Sous atmosphère inerte, 84 mg de cyanure de sodium, 597 mg d'oxyde de manganèse et 82 mg d'acide acétique glacial sont ajoutés à 1410 mg de 10-isopropyloxy-7,12-dioxabenzo[a]anthracèn-6-carbaldéhyde en solution dans 5 ml de méthanol anhydre. Le mélange réactionnel est porté à 40°C pendant 6 heures avant d'être filtré. Le filtrat est lavé à l'eau puis extrait au dichlorométhane. La phase organique est séchée sur sulfate de magnésium et concentrée sous vide. L'ester méthylique est purifié par chromatographie sur gel de silice (éluant : éther de pétrole/acétate d'éthyle : 9/1).
*Point de fusion : 128-130°C*

*Stade H : 10-Hydroxy-7,12-dioxa-benzo[a]anthracèn-6-carboxyméthyl ester*

Sous atmosphère inerte, 0,04 ml de tribromure de bore sont ajoutés à 50 mg de l'éther isopropylique (stade G) en solution dans 2 ml de dichlorométhane à -10°C. Après 15 minutes, le mélange réactionnel est hydrolysé. La phase aqueuse est extraite au dichlorométhane puis séchée sur sulfate de magnésium et concentrée sous vide. Le composé hydroxylé est purifié par chromatographie sur gel de silice (éluant : éther de pétrole/acétate d'éthyle : 7/3).
*Point de fusion : 168-170°C*

*Stade I : 10-Hydroxy-7,12-dioxa-benzo[a]anthracèn-6-(2-diméthylamino-éthyl) carboxamide, chlorhydrate*

On procède comme pour les exemples. 2 et 3.
*Point de fusion : décomposition à partir de 130°C*

**EXEMPLE 14 : 10-Méthoxy-7,12-dioxa-benzo[a]anthracèn-6-(2-diméthylaminoéthyl) carboxamide, chlorhydrate**

*Stade A : 10-Méthoxy-6-hydroxy-7,12-dioxa-benzo[a]anthracène*

On procède comme pour l'exemple 11.
*Point de fusion : 183-185°C*

*Stade B : 10-Méthoxy- 7,12-dioxa-benzo[a]anthracèn-6-carboxyméthylester*

On procède selon les stades D, E, F et G de l'exemple 13.
*Point de fusion : 108-110°C*

*Stade C : 10-Méthoxy-7,12-dioxa-benzo[a]anthracèn-6-(2-diméthylaminoéthyl)carboxamide, chlorhydrate*

On procède comme dans les exemples 2 et 3.
*Point de fusion : 213-215°C*

**EXEMPLE 15 : 7,12-dioxa-benzo[a]anthracèn-5-(2-diméthylamino-éthyl)carboxamide**

*Stade A : Benzo[1,4]dioxine*

Sous atmosphère inerte, 57,9 g de N-bromosuccinimide et 30 mg d'aza-iso-butyronitrile sont ajoutés à une solution de 20 g de 1,4-benzodioxane dans 300 ml de tétrachlorure de carbone. Le mélange réactionnel est porté à reflux, à l'aide d'une lampe, pendant 6 heures. Le succinimide formé est éliminé par filtration et le filtrat est concentré sous vide. Le produit obtenu est dissout dans 250 ml d'acétone et agité pendant 2 heures en présence de 108 g d'iodure de sodium. Après évaporation du solvant, le résidu vert est mis en solution dans un mélange eau/éther, la phase organique est ensuite lavée par une solution de thiosulfate de sodium à 20 % et séchée sur sulfate de magnésium. La benzodioxine est purifiée par chromatographie sur gel de silice.

*Stade B : 2-(1-Hydroxy-cyclohexan-1-yl)-benzo[1,4]dioxine*

Sous atmosphère inerte, 16,79 ml d'une solution de n-butyllithium 1,6M dans l'hexane sont ajoutés à une solution de 3 g de 1,4-benzodioxine dans 50 ml de THF anhydre à -78°C. Après 1 heure, 30 mmoles de cyclohexanone sont additionnées goutte à goutte. La température est maintenue à -78°C pendant 3 heures 30. Après hydrolyse et neutralisation du milieu réactionnel par une solution d'acide chlorhydrique à 10 %, la phase aqueuse est extraite à l'éther, puis la phase organique est séchée sur sulfate de magnésium et concentrée sous vide. L'alcool est purifié par chromatographie sur gel de silice (éluant : éther de pétrole/acétate d'éthyle) sous forme d'huile.

*Stade C : 2-(Cyclohexèn-1-yl)-benzo[1,4]dioxine*

Sous atmosphère inerte, 14 ml de triéthylamine et 3,85 ml de chlorure de mésyle sont additionnés lentement à une solution de 10 ml de 2-(1-hydroxy-cyclohexan-1-yl)-benzo[1,4]dioxine dans 75 ml de dichlorométhane à 0°C. Le mélange est porté à reflux après 15 minutes pendant 1,5 heures. Après hydrolyse, une extraction au dichlorométhane est réalisée, puis la phase organique est séchée sur sulfate de magnésium et concentrée sous vide. Le diène est purifié par chromatographie sur gel de silice (éluant : éther de pétrole/acétate d'éthyle : 7/3) sous forme d'huile.

*Stade D : 1,2,3,4,4a,6a-Hexahydro-7,12-dioxa-benzo[a]anthracène-5-carboxylate de méthyle*

Dans un tube scellé, 500 mg de 2-(cyclohexèn-1-yl)-benzo[1,4]dioxine et 1,4 ml de propiolate de méthyle sont agités 22 heures 30 minutes à 70°C. Les régioisomères sont séparés par chromatographie sur gel de silice (éluant : éther de pétrole/acétate d'éthyle : 98/2).
*Point de fusion : 72-74°C*

*Stade E : 7,12-dioxa-benzo[a]anthracène-5-carboxylate de méthyle*

Sous atmosphère inerte, 370 mg de 1,2,3,4,4a,6a-hexahydro-7,12-dioxa-benzo[a]anthracène-5-carboxylate de méthyle en solution dans 10 ml de toluène sont portés à reflux pendant 6 heures en présence de 986 mg de DDQ. Après refroidissement, le mélange réactionnel est lavé avec une solution de soude à 8 %. La phase organique est séchée sur sulfate de magnésium puis concentrée sous vide. La purification est effectuée par chromatographie sur gel de silice (éluant : éther de pétrole/acétate d'éthyle : 95/5).
*Point de fusion : 144-146°C*

*Stade F : 7,12-Dioxa-benzo[a]anthracène-5-(2-diméthylamino-éthyl)carboxamide*

On procède comme pour l'exemple 2.
*Point de fusion : 198-200°C*

**EXEMPLE 16: 7,12-Dioxa-benzo[a]anthracène-6-(2-diméthylamino-éthyl)carboxamide**

On procède comme dans l'exemple 15 en choisissant l'autre régioisomère au stade D.
*Point de fusion : 139-141°C*

**EXEMPLE 17 : N2-(7,12-Dioxa-benzo[a]anthracène-5,6-maléimido)-N1,N1-diméthyléthylènediamine, chlorhydrate**

*Stade A : 7,12-Dioxa-benzo[a]anthracène-5,6-dicarboxylate de méthyle*

On procède selon les stades A, B, C, D et E de l'exemple 15 en utilisant l'acétylène dicarboxylate de méthyle à la place du propiolate de méthyle dans le stade D.
*Point de fusion : 182-184°C*

*Stade B : N2-(7,12-Dioxa-benzo[a]anthracène-5,6-maléimido)-N1,N1-diméthyl-éthylène diamine*

Sous atmosphère inerte, 600 mg de 7,12-dioxa-benzo[a]anthracène-5,6-dicarboxylate de méthyle sont portés à 100°C dans 10 ml de N,N-diméthyléthylènediamine pendant 24 heures. Le composé obtenu est purifié par chromatographie sur gel de silice (éluant : acétate d'éthyle/méthanol : 85/15).
*Point de fusion : 186-188°C*

*Stade C : N2-(7,12-dioxa-benzo[a]anthracène-5,6-maléimido)-N1,N1-diméthyl-éthylène diamine, chlorhydrate*

On procède comme dans l'exemple 3.
*Point de fusion : 208-210°C*

**EXEMPLE 18 : N2-(7,12-Dioxa-3-aza-benzo[a]anthracène-5,6-maléimido)-N1,N1-diméthyléthylènediamine, chlorhydrate**

*Stade A : 1,2,4,4a,6a-Pentahydro-7,12-dioxa-3-aza-benzo[a]anthracène-3-carboxylate d'éthyle-5,6-dicarboxylate de méthyle*

On procède selon les stades A, B, C et D de l'exemple 15 en remplaçant la cyclohexanone par la N-carbéthoxy pipéridin-4-one dans le stade B et le propiolate de méthyle par l'acétylène dicarboxylate de méthyle dans le stade D.

*Stade B : 1,2,4-Trihydro-7,12-dioxa-3-aza-benzo[a]anthracène-3-carboxylate* d'éthyle-5,6-*dicarboxylate de méthyle*

Sous atmosphère inerte, 1,5 g de 1,2,4,4a,6a-pentahydro-7,12-dioxa-3-aza-benzo[a]anthracène-3-carboxylate d'éthyle-5,6-dicarboxylate de méthyle sont dissous dans 25 ml de toluène. La solution est portée à 35°C après addition de 1,196 g d'ortho-chloranil pendant 48 heures. Après refroidissement, le mélange réactionnel est lavé avec une solution de soude à 8 %. La phase organique est séchée sur sulfate de magnésium puis concentrée sous vide. La purification est effectuée par chromatographie sur gel de silice (éluant : éther de pétrole/acétate d'éthyle : 6/4).
*Point de fusion : 153-155°C*

*Stade C : 7,12-Dioxa-3-aza-benzo[a]anthracène-5,6-dicarboxylate de méthyle*

Sous atmosphère inerte, 1 g de 1,2,4-trihydro-7,12-dioxa-3-aza-benzo[a]anthracène-3-carboxylate d'éthyle-5,6-dicarboxylate de méthyle est porté à reflux dans 25 ml de toluène en présence de 1,574 g d'ortho-chloranil pendant 48 heures. On procède ensuite comme pour le stade B.
*Point de fusion : 160-162°C*

*Stade D : N2-(7,12-Dioxa-3-aza-benzo[a]anthracène-5,6-maléimido)-N1,N1-diméthyléthylènediamine, chlorhydrate*

On procède comme pour les stades B et C de l'exemple 17.
*Point de fusion: 278-280°C*

**EXEMPLE 19 : N2-[7,12-Dioxa-3-(2-hydroxy-éthoxy)-benzo[a]anthracène-5,6-maléimido]-N1,N1-diméthyléthylènediamine, chlorhydrate**

*Stade A : 7,12-Dioxa-3-(2-hydroxy-éthoxy)-benzo[a]anthracène-5,6-dicarboxylate de méthyle*

On procède comme pour les stades A, B, C, D et E de l'exemple 15 en remplaçant la cyclohexanone par la 1,4-cyclohexane-dione monoéthylène acétal dans le stade B et le propiolate de méthyle par l'acétylène dicarboxylate de méthyle dans le stade D.
*Point de fusion : 182-184°C*

*Stade B : N2-[7,12-Dioxa-3-(2-hydroxy-éthoxy)-benzo[a]anthracène-5,6-maléimido]-N1,N1-diméthyléthylènediamine, chlorhydrate*

On procède comme pour les stades B et C de l'exemple 17.
*Point de fusion : 298-300°C*

**EXEMPLE 20 : N2-(7,12-Dioxa-3-hydroxy-benzo[a]anthracène-5,6-maléimido)-N1,N1-diméthyléthylènediamine, chlorhydrate**

*Stade A : 1,2,4-Trihydro- 7,12-dioxa-3-(éthylèneacétal)-benzo[a]anthracène-5,6-dicarboxylate de méthyle*

On procède comme pour le stade A de l'exemple 19.
*Point de fusion : 144-146°C*

*Stade B : 1,2,4-Trihydro-7,12-dioxa-3-oxo-benzo[a]anthracène-5,6-dicarboxylate de méthyle*

Sous atmosphère inerte, 1 g d'acétal est porté à reflux d'acétone en présence de 609 mg d'ester paratoluènesulfonique. Après 72 heures, le solvant est évaporé sous vide et le mélange réactionnel est lavé à l'eau puis extrait à l'acétate d'éthyle. La phase organique est séchée sur sulfate de magnésium et concentrée sous vide. La cétone est purifiée par chromatographie sur gel de silice (éluant : éther de pétrole/acétate d'éthyle : 6/4).
*Point de fusion : 190-192°C*

*Stade C : 1,2-Dihydro-7,12-dioxa-3-(tertbutyldiméthylsilyléther)-benzo[a]anthracène-5,6-dicarboxylate de méthyle*

Sous atmosphère inerte, 1 ml de diisopropylamidure de lithium et 0,3 ml de tétraméthyléthylènediamine sont additionnés goutte à goutte successivement à 500 mg de cétone (stade B) solubilisée dans 10 ml de tétrahydrofurane et 0,5 ml d'hexaméthylphosphoramide à -78°C. Le mélange est maintenu à -78°C pendant 2 heures avant l'addition de 415 mg de chlorure de diméthyl-tertbutylsilane dissous dans un minimum de tétrahydrofurane. Après 3 heures, le mélange réactionnel est ramené à -10°C puis les produits volatiles sont évaporés sous pression réduite. L'éther silylé est purifié par chromatographie éclair sur gel de silice traité par la triéthylamine (éluant : éther de pétrole/acétate d'éthyle 9/1).
*Point de fusion : 98-100°C*

*Stade D : 7,12-Dioxa-3-(tertbutyldiméthylsilyléther)-benzo[a]anthracène-5,6-dicarboxylate de méthyle*

On procède comme pour le stade E de l'exemple 15.
*Point de fusion : 68-70°C*

*Stade E : 3-Hydroxy-7, 12-dioxa-benzo[a]anthracène-5,6-dicarboxylate de méthyle*

Sous atmosphère inerte, 450 mg d'éther silylé (stade D) sont dissous dans du tétrahydrofurane avant l'addition, à température ambiante, de 1,9 ml de fluorure de tétra-tertbutylammonium. Après 30 minutes, le mélange réactionnel est lavé à l'eau. La phase aqueuse est extraite au dichlorométhane, la phase organique séchée sur sulfate de magnésium et concentrée sous vide. Le composé hydroxylé est purifié par chromatographie sur gel de silice (éluant : éther de pétrole/acétate d'éthyle : 5/5).
*Point de fusion : 198-200°C*

*Stade F : N2-(7,12-Dioxa-3-hydroxy-benzo[a]anthracène-5,6-maléimido)-N1,N1-diméthyléthylènediamine, chlorhydrate*

On procède comme pour les stades B et C de l'exemple 17.
*Point de fusion : 298-300°C*

**EXEMPLE 21 : N2-(7,12-Dioxa-3-méthoxy-benzo[a]anthracène-5,6-maléimido)-N1,N1-diméthyléthylènediamine, chlorhydrate**

*Stade A : 3-Hydroxy-7,12-dioxa-benzo[a]anthracène-5,6-dicarboxylate de méthyle*

On procède comme pour les stades A, B, C, D et E de l'exemple 20.

*Stade B : 3-Méthoxy-7,12-dioxa-benzo[a]anthracène-5,6-dicarboxylate de méthyle*

On procède comme dans le stade F de l'exemple 7.
*Point de fusion : 170-172°C*

*Stade C : N2-(7,12-Dioxa-3-méthoxy-benzo[a]anthracène-5,6-maléimido)-N1,N1-diméthyléthylènediamine, chlorhydrate*

On procède comme pour les stades B et C de l'exemple 17.
*Point de fusion : 258-260°C*

**EXEMPLE 22 : N2-(7,12-Dioxa-3-benzyloxy-benzo[a]anthracène-5,6-maléimido)-N1,N1-diméthyléthylènediamine, chlorhydrate**

On procède comme pour l'exemple 21 en remplaçant le stade F de l'exemple 7 par la procédure de l'exemple 8.

**EXEMPLE 23 : 7,12-Dioxa-3-hydroxy-benzo[a]anthracène-5,6-(2-diméthylaminoéthyl)carboxamide, chlorhydrate**

On procède comme pour l'exemple 20.
*Point de fusion : décomposition à 160°C*

**EXEMPLE 24 : 7,12-dioxa-benzo[a]anthracène-6-(1,3-dihydroxy-3-méthylpropyl) carboxamide**

*Stade A : 7,12-dioxa-benzo[a]anthracène-6-carboxylate de méthyle*

On procède comme dans l'exemple 15 jusqu'au stade E en choisissant l'autre régioisomère au stade D.

*Stade B : 7,12-dioxa-benzo[a]anthracène-6-(1,3-dihydroxy-3-méthylpropyl)carboxamide*

Sous atmosphère inerte, 70 mg de 7,12-dioxa-benzo[a] anthracène-6-carboxylate de méthyle dissous dans 3 ml de tétrahydrofurane sont portés au reflux en présence de 251 mg de 2-méthyl-2-amino-propane-1,3-diol, pendant 76 heures. Le composé est purifié par chromatographie sur gel de silice neutralisé par de la triéthylamine (éluant : acétate d'éthyle/méthanol : 9/1).
*Point de fusion : 136-138 °C*

**EXEMPLE 25 : 7,12-dioxa-benzo[a]anthracène-5-(2-diméthylamino-éthyl)méthylamine, chlorhydrate**

*Stade A : 7,12-dioxa -benzo[a]anthracène-5-carboxylate de méthyle*

On procède comme pour les stades A, B, C, D, E et F de l'exemple 15.

*Stade B : 7,12-dioxa-benzo[a]anthracène-5-yl-méthanol*

Sous atmosphère inerte, 72 mg d'hydrure de lithium-aluminium et 170 mg du composé du stade A dissous dans 5 ml de tétrahydrofurane, sont agités à température ambiante pendant 45 minutes. Le mélange réactionnel est alors hydrolysé, la phase aqueuse est extraite avec de l'acétate d'éthyle. La phase organique est séchée sur sulfate de magnésium et concentrée sous vide. Le composé est purifié par chromatographie éclair sur gel de silice (éluant : éther de pétrole/acétate d'éthyle : 7/3).
*Point de fusion : 189-191 °C*

*Stade C : 7,12-dioxa-benzo[a]anthracène-5-carbaldéhyde*

130 mg d'alcool et 642 mg d'oxyde de manganèse, dissous dans 10 ml de dichlorométhane, sont agités 3 heures à température ambiante, puis la solution est filtrée. Le filtrat est concentré sous vide et purifié par chromatographie sur gel de silice (éluant : éther de pétrole/acétate d'éthyle : 9/1).
*Point de fusion : 133-135 °C*

*Stade D : 7,12-dioxa-benzo[a]anthracène-5-(2-diméthylamino-éthyl)méthylamine*

Sous atmosphère inerte, 34 mg de chlorure de zinc et 32 mg de cyanoborohydrure de sodium dissous dans 1 ml de méthanol sont additionnés à une solution, dans 5 ml de méthanol, de 130 mg d'aldéhyde et de 0,22 ml de diméthyléthylène diamine. Après 6 heures à 35 °C, le méthanol est évaporé. Le résidu est repris dans le dichlorométhane, et lavé à l'eau. La phase organique est séchée sur sulfate de magnésium et concentrée sous vide. Le composé est purifié par chromatographie sur gel de silice traité par de la triéthylamine (éluant : acétate d'éthyle/méthanol : 9/1).

*Stade E: 7,12-dioxa-benzo[a]anthracène-5-(2-diméthylamino-éthyl) méthylamine, chlorhydrate*

On procéde comme dans l'exemple 3.

*Point de fusion : 254-256 °C*

**EXEMPLE 26 : 7,12-dioxa-benzo[a]anthracène-6-(1,3-dihydroxy-3-méthylpropyl) méthylamine**

On procède comme pour les stades A, B, C, D et E de l'exemple 15 pour obtenir le 7,12-dioxa-benzo[a] anthracène-6-carboxylate de méthyle que l'on traite selon les procédés des stades B, C et D de l'exemple 25 en utilisant comme amine dans l'étape D, la 2-méthyl-2-amino-propane-1,3-diol.
*Point de fusion : 148-150 °C.*

**EXEMPLE 27 : Iodure de 7,12-dioxa-5-aza-5-méthyl-benzo[a]anthracène**

On procède comme dans l'exemple 1 des stades A, B, C, D, E, F et G et l'on traite les 140 mg de 7,12-dioxa-5-aza-benzo[a]anthracène par 1,85 ml d'iodure de méthyle. Après 12 heures à température ambiante à l'abri de la lumière, le produit précipité est isolé par filtration.
*Point de fusion : 263-265 °C.*

**EXEMPLE 28 : N2-(8-méthoxy-7,12-dioxa-5-aza-benzo[a]anthracène-6-yl)-N1, N1-diméthyléthane-1,2-diamine, chlorhydrate**

On procède comme pour les stades A, B, C, D, E et F de l'exemple 7 puis A, B, C, D, E et F de l'exemple 1 en prenant comme substrat de départ le 8-méthoxy-benzo[1,4]-dioxine-2-carboxylate d'éthyle pour conduire au dérivé chloré en position 6 correspondant que l'on traite selon la procédure de l'exemple 2 pour obtenir la diamine, et le chlorhydrate correspondant est obtenu en procédant comme dans l'exemple 3.
*Point de fusion : 256-258 °C*

**EXEMPLE 29 : N2-(8-hydroxy-7,12-dioxa-5-aza-benzo[a]anthracène-6-yl)-N1,N1-diméthyléthane-1,2-diamine, chlorhydrate**

On procède comme pour les stades A, B et C de l'exemple 10 en prenant comme substrat de départ pour le stade A le 8-méthoxy-benzo[1,4]-dioxine-2-carboxylate d'éthyle.
*Point de fusion : 286-288 °C*

**EXEMPLE 30 : N2-(11-méthoxy-7,12-dioxa-5-aza-benzo[a]anthracène-6-yl)-N1, N1-diméthyléthane-1,2-diamine, chlorhydrate**

On procède comme dans l'exemple 28 en prenant comme substrat de départ le 5-méthoxybenzo[1,4]-dioxine-2-carboxylate d'éthyle.
*Point de fusion : 271-273 °C*

**EXEMPLE 31 : N2-[11-hydroxy-7,12-dioxa-5-aza-benzo[a]anthracène-6-yl)-N1,N1-méthyléthane-1,2-diamine, chlorhydrate**

On procéde comme dans l'exemple 29 en prenant comme substrat de départ le 5-méthoxybenzo[1,4]-dioxine-2-carboxylate d'éthyle.
*Point de fusion : 265-267 °C*

**ETUDE PHARMACOLOGIOUE**

Les exemples suivants permettent de mettre en évidence les propriétés cytotoxiques des composés de l'invention, leur action sur le cycle cellulaire et *in vivo* sur un modèle leucémique.

**EXEMPLE A : Cytotoxicité des composés**

Trois lignées cellulaires ont été utilisées :

- 1 leucémie murine, L1210,
- 1 carcinome épidermoïde humain, KB-3-1,
- 1 carcinome pulmonaire humain, non à petites cellules, A549.

Les cellules sont cultivées dans du milieu de culture RPMI 1640 complet contenant 10 % de sérum de veau foetal, 2 mM de glutamine, 50 unités/ml de pénicilline, 50 μg/ml de streptomycine et 10 mM d'Hépès.
Les cellules sont réparties dans des microplaques et exposées aux composés cytotoxiques. Elles sont ensuite incubées pendant 2 jours (L1210) et 4 jours (A549, KB-3-1). Le nombre de cellules viables est ensuite quantifié par un essai colorimétrique, le Minoculture Tetrazolium Assay (Carmichael J., De Graff W. G., Gazdar A.F., Minna J.D. and Mitchell J.R., Evaluation of a tetrazolium-based semi-automated colorimetric assay : assessment of chemosensitivity testing, Cancer Res., 47, 936-942, 1987). Les résultats sont exprimés en $IC_{50}$, concentration en cytotoxique qui inhibe à 50 % la prolifération des cellules traitées.
Les résultats obtenus montrent une bonne cytotoxicité générale sur la lignée L1210 avec des $IC_{50}$ comprises entre 0,2 et 1 μM. Par ailleurs des propriétés cytotoxiques vis-à-vis de tumeurs solides comme la lignée KB-3-1 et la lignée A549 ont pu être mises en évidence.

**EXEMPLE B : Action sur le cycle cellulaire**

Les cellules L1210 sont incubées pendant 21 heures à 37°C en présence de différentes concentrations en produits testés. Les cellules sont ensuite fixées par de l'éthanol à 70 % (V/V), lavées 2 fois dans du PBS et incubées 30 minutes à 20°C dans du PBS contenant 100 μg/ml de RNAse et 50 μg/ml de iodure de propidium. Le pourcentage en phase G2+M est calculé et les résultats sont exprimés suivant une classification déterminée en fonction du pourcentage des cellules accumulées en phase G2+M après 21 heures par rapport au témoin (témoin : 20 %). Les composés de l'invention montrent une accumulation à plus de 70 % des cellules en phase G2+M après 21 heures pour des concentrations en produits allant de 0,5 à 50 μM.

**EXEMPLE C : Activité *in vivo* : activité antitumorale sur la leucémie P388**

La lignée P388 (leucémie murine) a été fournie par le National Cancer Institute (Frederick, USA). Les cellules tumorales ($10^6$ cellules) ont été inoculées au jour 0 dans la cavité péritonéale de souris BDFI femelles (Iffer-Credo, France) pesant de 18 à 20 g (groupes de 8 à 10 animaux). Les produits ont été administrés au jour 1 aux doses indiquées par voie intrapéritonéale. L'activité antitumorale est exprimée en pourcentage de T/C:

$$\% \text{ T/C} = \frac{\text{Temps de survie médian des animaux traités}}{\text{Temps de survie médian des animaux contrôles}} \times 100$$

Les résultats obtenus montrent une excellente activité in *vivo* sur le modèle leucémique P388 avec par exemple un T/C de 196 % pour une dose de 50 mg/kg, ainsi qu'une faible cytotoxicité des composés témoin d'un excellent index thérapeutique.

**EXEMPLE D : Comprimés dosés à 10 mg de N2-(7,12-dioxa-3-hydroxybenzo[a]anthracène-5,6-maléimido)-N1,N1-diméthyléthylènediamine, chlorhydrate (Exemple 20)**

| Formule de préparation pour 1000 comprimés : | |
|---|---|
| N2-(7,12-dioxa-3-hydroxy-benzo[a]anthracène-5,6-maléimido)-N1,N1-diméthyléthylènediamine | 10 g |
| Amidon de blé | 15 g |
| Amidon de maïs | 15 g |
| Lactose | 65 g |
| Stéarate de Mg | 2 g |
| Silice | 1 g |
| Hydroxypropylcellulose | 2 g |

**Revendications**

1. Composés de formule (I) :

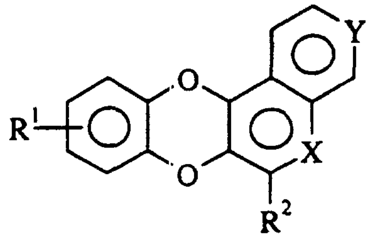

(I)

dans laquelle :

$R^1$ représente un atome d'hydrogène ou un groupement de formule O-R, dans lequel R représente un atome d'hydrogène, un groupement alkyle ($C_1$-$C_6$) linéaire ou ramifié, éventuellement substitué par un groupement aryle ou hétéroaryle,

$R^2$ représente un atome d'hydrogène, un atome d'halogène, un groupement hydroxy, alkoxy ($C_1$-$C_6$) linéaire ou ramifié, formyle, $CF_3SO_3$, cyano, alkoxycarbonyle ($C_1$-$C_6$) linéaire ou ramifié, aryloxycarbonyle, NR'aR'b dans lequel R'a représente un groupement dialkylaminoalkyle ($C_1$-$C_6$) (chaque partie alkyle étant constituée par une chaine comportant de 1 à 6 atomes de carbone, linéaire ou ramifiée, identique ou différente indépendamment l'une de l'autre), et R'b représente un atome d'hydrogène ou un groupement alkyle ($C_1$-$C_6$) linéaire ou ramifié, ou un groupement BNR"aR"b dans lequel B représente un groupement carbonyle ou méthylène, R"a et R"b ont respectivement la même définition que R'a et R'b ou R"a représente un groupement alkyle ($C_1$-$C_6$) linéaire ou ramifié substitué par au moins un groupement hydroxy,

X représente un atome d'azote (éventuellement substitué par un groupement R'c alkyle ($C_1$-$C_6$) linéaire ou ramifié), ou un groupement C-$R^3$ dans lequel $R^3$ représente un atome d'hydrogène, un groupement alkoxycarbonyle ($C_1$-$C_6$) linéaire ou ramifié, ou un groupement BNR"aR"b dans lequel B représente un carbonyle ou un groupement méthylène et R"a et R"b ont la même définition que précédemment,
ou $R^2$ et X, lorsque X représente un groupement C-$R^3$, avec l'atome de carbone qui les porte forment ensemble un cycle de formule (II) :

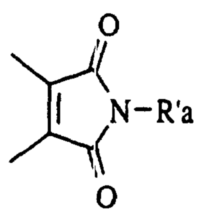

(II)

dans laquelle R'a a la même définition que précédemment,

Y représente un atome d'azote ou un groupement C-$R^4$ dans lequel $R^4$ représente un atome d'hydrogène, ou un groupement de formule O-R" dans laquelle R" représente un atome d'hydrogène, un groupement alkyle ($C_1$-$C_6$) linéaire ou ramifié (éventuellement substitué par un groupement hydroxy), un groupement aryle ou hétéroaryle,

étant entendu que lorsque X représente un atome d'azote et Y un groupement CH, ou lorsque X représente un groupement CH et Y un atome d'azote ou lorsque X et Y représentent simultanément un groupement CH alors $R^1$ et $R^2$ ne peuvent représenter simultanément un atome d'hydrogène,
étant entendu que le terme aryle désigne un groupement phényle ou naphtyle éventuellement substitué de façon identique ou différente par un ou plusieurs atomes d'halogène ou groupements alkyle ($C_1$-$C_6$) linéaire ou ramifié, hydroxy, alkoxy ($C_1$-$C_6$) linéaire ou ramifié ou trihalogénométhyle,
et le terme hétéroaryle désigne un groupement aromatique mono ou bicyclique contenant 1 ou 2 hétéroatomes choisis parmi O, S, N éventuellement substitué de façon identique ou différente par un ou plusieurs atomes d'halogène ou groupements alkyle ($C_1$-$C_6$) linéaire ou ramifié, hydroxy, alkoxy ($C_1$-$C_6$) linéaire ou ramifié ou trihalogénométhyle,

leurs isomères ainsi que leurs sels d'addition à un acide ou à une base pharmaceutiquement acceptable.

**2.** Composés de formule (I) selon la revendication 1 pour lesquels $R^2$ représente un groupement NR'aR'b dans lequel R'a représente un groupement dialkyle ($C_1$-$C_6$) -linéaire ou ramifié- aminoalkyle ($C_1$-$C_6$) linéaire ou ramifié, chaque partie alkyle étant identique ou différente indépendamment l'une de l'autre, et R'b représente un atome d'hydrogène ou un groupement alkyle ($C_1$-$C_6$) linéaire ou ramifié, leurs isomères ainsi que leurs sels d'addition à un acide ou à une base pharmaceutiquement acceptable.

**3.** Composés de formule (I) selon la revendication 1 pour lesquels $R^2$ représente un groupement BNR"aR"b dans lequel B représente un carbonyle ou un groupement méthylène, R"a représente un groupement dialkyle ($C_1$-$C_6$) -linéaire ou ramifié- aminoalkyle ($C_1$-$C_6$) linéaire ou ramifié, chaque partie alkyle étant identique ou différente indépendamment l'une de l'autre, et R"b représente un atome d'hydrogène ou un groupement alkyle ($C_1$-$C_6$) linéaire ou ramifié, leurs isomères ainsi que leurs sels d'addition à un acide ou à une base pharmaceutiquement acceptable.

**4.** Composés de formule (I) selon la revendication 1 pour lesquels X représente un groupement C-$R^3$ dans lequel $R^3$ représente un groupement BNR"aR"b dans lequel B représente un carbonyle ou un groupement méthylène, R"a représente un groupement dialkyle ($C_1$-$C_6$) -linéaire ou ramifié- aminoalkyle ($C_1$-$C_6$) linéaire ou ramifié, chaque partie alkyle étant identique ou différente indépendamment l'une de l'autre, et R"b représente un atome d'hydrogène ou un groupement alkyle ($C_1$-$C_6$) linéaire ou ramifié, leurs isomères ainsi que leurs sels d'addition à un acide ou à une base pharmaceutiquement acceptable.

**5.** Composés de formule (I) selon la revendication 1 pour lesquels $R^2$ et X, lorsque X représente un groupement C-$R^3$, forment ensemble avec l'atome de carbone qui les porte un cycle de formule (II) :

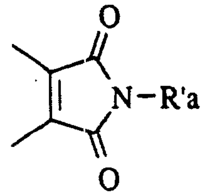

(II)

dans laquelle R'a représente un groupement dialkyle ($C_1$-$C_6$) -linéaire ou ramifié- aminoalkyle ($C_1$-$C_6$), linéaire ou ramifié, chaque partie alkyle étant identique ou différente indépendamment l'une de l'autre, leurs isomères ainsi que leurs sels d'addition à un acide ou à une base pharmaceutiquement acceptable.

**6.** Composés de formule (I) selon la revendication 5 pour lesquels R'a représente un groupement diméthylaminoéthyle, leurs isomères ainsi que leurs sels d'addition à un acide ou à une base pharmaceutiquement acceptable.

**7.** Composé selon la revendication 1 qui est le N2-(7,12-dioxa-3-hydroxy-benzo[a]anthracène-5,6-maléimido)-N1,N1-diméthyléthylènediamine, chlorhydrate et ses sels d'addition à une base ou à un acide pharmaceutiquement acceptable.

**8.** Procédé de préparation des composés de formule (I) **caractérisé en ce que** l'on utilise comme produit de départ :

- *pour les composés où X représente un atome d'azote ou un groupement CH,* le composé de formule (III) :

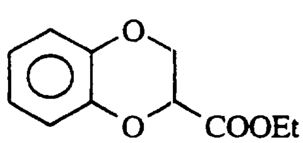

(III)

qui est - soit dibromé puis traité à l'acide métachloroperbenzoïque et soumis à l'action du l'iodure de potassium pour obtenir le composé (IIIa):

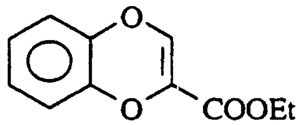

(IIIa)

- soit acylé selon une réaction de Friedel Crafts, dibromé puis traité à l'acide métachloroperbenzoïque, pour conduire au composé de formule (IV):

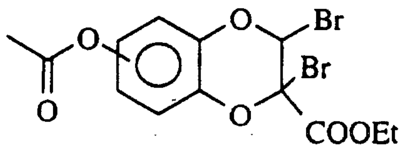

(IV)

qui, soumis à l'action de l'iodure de sodium, puis d'une base telle que l'éthylate de sodium, permet d'obtenir le composé (V) :

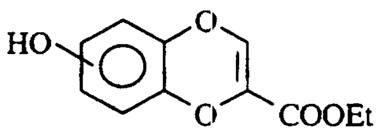

(V)

sur lequel on condense les électrophiles de formule R'''-Halogène, dans laquelle R''' représente un groupement alkyle ($C_1$-$C_6$) linéaire ou ramifié, éventuellement substitué par un groupement aryle ou hétéroaryle, pour conduire au composé de formule (VI) :

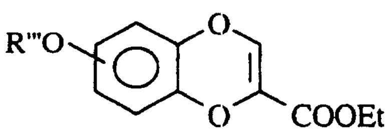

(VI)

dans laquelle R''' a la même définition que précédemment,
l'ensemble des composés (IIIa) et (VI) formant le composé de formule (VII) :

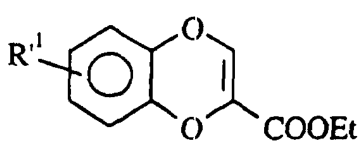

(VII)

dans laquelle $R'^1$ représente un atome d'hydrogène, un groupement alkoxy ($C_1$-$C_6$) linéaire ou ramifié, éventuellement substitué par un groupement aryle ou hétéroaryle,
sur lequel on condense, après saponification par l'hydroxyde de sodium aqueux par exemple, la diéthylamine en présence d'un agent de couplage comme le 1,3-diméthylaminopropyl-3-éthylcarbodiimide, pour conduire au composé de formule (VIII) :

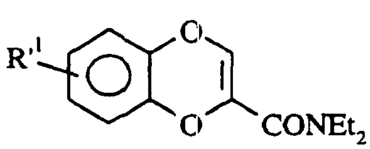

(VIII)

dans laquelle R'[1] a la même signification que précédemment,
qui est soumis à l'action d'une base forte, puis au chlorure de triméthylétain, pour conduire au composé de formule (IX) :

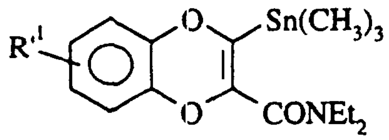

(IX)

dans laquelle R'[1] a la même signification que précédemment,
sur lequel on condense, en présence d'iodure de cuivre et d'un complexe de métal de transition comme le palladium :

- **soit** la N-tert-butoxycarbonyl-2-iodoaniline, pour obtenir le composé de formule (X) :

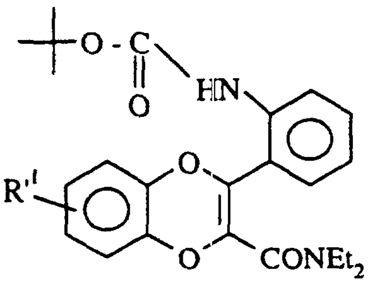

(X)

dans laquelle R'[1] a la même définition que précédemment,
qui conduit après hydrolyse acide au composé de formule (XI) :

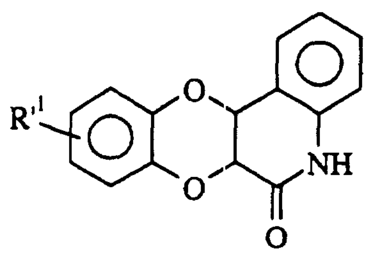

(XI)

dans laquelle R'[1] a la même définition que précédemment,
sur lequel on fait agir $POCl_3$, pour obtenir le composé de formule (I/a), cas particulier des composés de formule (I):

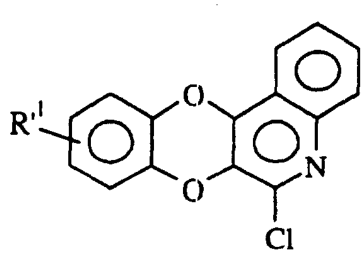

(I/a)

dans laquelle R'[1] est défini comme précédemment,
que l'on soumet, dans le cas où R'[1] représente un groupement alkoxy ($C_1$-$C_6$) linéaire ou ramifié à un agent

de déalkylation comme le tribromure de bore par exemple, pour conduire au composé de formule (I/b), cas particulier des composés de formule (I):

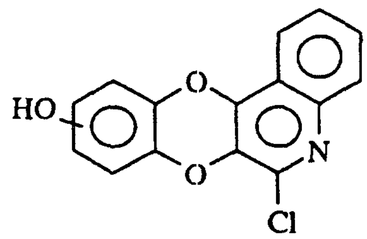

(I/b)

l'ensemble des composés (I/a) et (I/b) formant le composé de formule (I/c), cas particulier des composés de formule (I) :

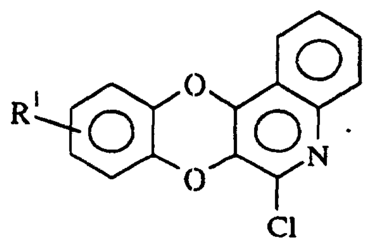

(I/c)

dans laquelle $R^1$ a la même signification que dans la formule (I), composé de formule (I/c) :

* que l'on soumet à un agent réducteur comme le zinc, pour conduire au composé de formule (I/d), cas particulier des composés de formule (I) :

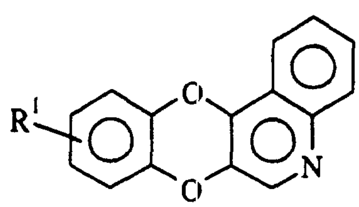

(I/d)

dans laquelle $R^1$ a la même signification que précédemment, sur lequel on effectue une N-alkylation par action d'un agent alkylant comme l'iodure de méthyle pour conduire au composé de formule (I/e), cas particulier des composés de formule (I):

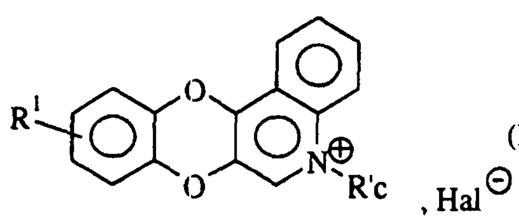

(I/e)

dans laquelle R'c représente un groupement alkyle ($C_1$-$C_6$) linéaire ou ramifié et Hal un halogène,

* ou, sur lequel on condense les diamines de formule HNR'aR'b où R'a représente un groupement dialkyle ($C_1$-$C_6$) -linéaire ou ramifié - aminoalkyle ($C_1$-$C_6$) linéaire ou ramifié, chaque partie alkyle étant identique ou différente indépendamment l'une de l'autre, et R'b représente un atome d'hydrogène ou un groupement

alkyle ($C_1$-$C_6$) linéaire ou ramifié, pour conduire au composé de formule (I/f), cas particulier des composés de formule (I):

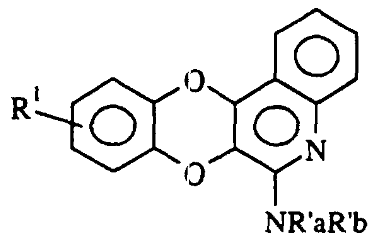

(I/f)

dans laquelle $R^1$, R'a et R'b ont la même signification que précédemment,

- **soit** le 2-iodotoluène pour obtenir le composé de formule (XII) :

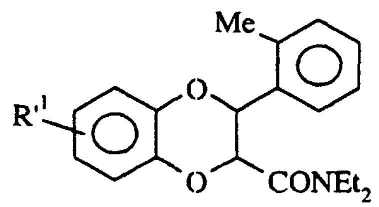

(XII)

dans laquelle $R'^1$ a la même signification que précédemment,
qui conduit, après l'action d'une base forte comme le diisopropylamidure de lithium au composé de formule (I/g), cas particulier des composés de formule (I) :

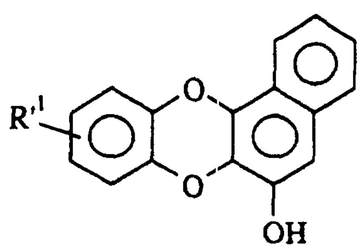

(I/g)

dans laquelle $R'^1$ est tel que défini précédemment,
composé de formule (I/g) que l'on soumet :

* dans le cas où $R'^1$ représente un groupement alkoxy ($C_1$-$C_6$)linéaire ou ramifié à un agent de déalkylation comme le tribromure de bore par exemple, pour conduire au composé de formule (I/h), cas particulier des composés de formule (I) :

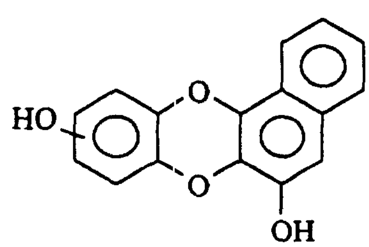

(I/h)

* ou à l'action successive de l'anhydride trifluorométhanesulfonique, d'un cyanure en présence d'un complexe de métal de transition comme le palladium, d'un agent réducteur comme l'hydrure de diisobutyl

aluminium, pour obtenir le composé de formule (I/i), cas particulier des composés de formule (I) :

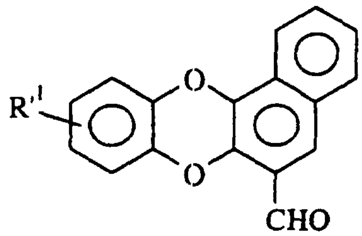

(I/i)

dans laquelle R'[1] a la même définition que précédemment, qui est oxydé en ester méthylique par l'action d'un agent oxydant comme l'oxyde de manganèse en présence de méthanol, pour conduire au composé de formule (I/j), cas particulier des composés de formule (I):

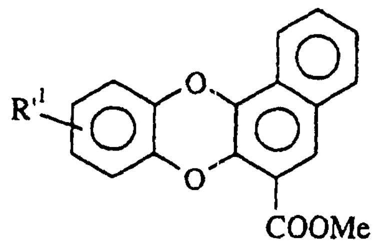

(I/j)

dans laquelle R'[1] a la même définition que précédemment, sur lequel on fait agir, dans le cas où R'[1] représente un groupement alkoxy ($C_1$-$C_6$)linéaire ou ramifié, un agent de déalkylation comme le tribromure de bore, pour conduire au composé de formule (I/k), cas particulier des composés de formule (I) :

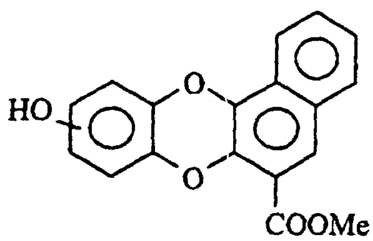

(I/k)

l'ensemble des composés (I/j) et (I/k) formant le composé de formule (I/l), cas particulier des composés de formule (I):

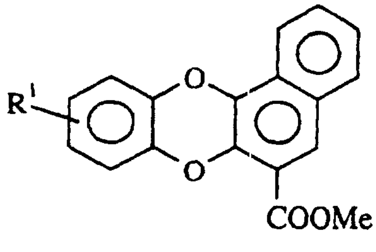

(I/l)

dans laquelle R[1] a la même définition que dans la formule (I), sur lequel on condense les diamines de formule HNR'aR'b où R'a représente un groupement dialkyle ($C_1$-$C_6$) -linéaire ou ramifié- aminoalkyle ($C_1$-$C_6$) linéaire ou ramifié, (chaque partie alkyle étant identique ou différente) et R'b représente un atome d'hydrogène ou un groupement alkyle ($C_1$-$C_6$) linéaire ou ramifié, pour conduire au composé de formule (I/m), cas particulier des composés de formule (I) :

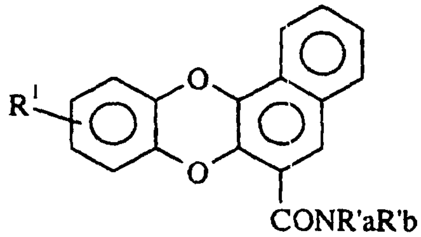

(I/m)

dans laquelle $R^1$, R'a et R'b ont la même signification que précédemment, étant entendu que les composés (I/m) peuvent être obtenus à partir du composé (I/k) soumis aux électrophiles de formule $R'^1$-Halogène où $R'^1$ est tel que défini précédemment, en milieu basique,

- *pour les composés où X ne représente pas un atome d'azote,* le composé de formule (XIII) :

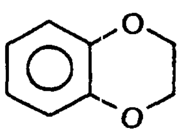

(XIII)

qui est :

- soit dibromé puis traité à l'acide métachloroperbenzoïque et soumis à l'action de l'iodure de potassium pour obtenir le composé (XIIIa) :

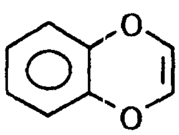

(XIIIa)

- soit acylé selon une réaction de Friedel Crafts, dibromé, traité à l'acide métachloroperbenzoique, soumis à l'action de l'iodure de sodium puis d'une base pour obtenir le composé (XIV):

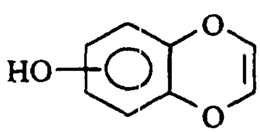

(XIV)

sur lequel on condense les électrophiles de formule R'''-Halogène dans laquelle R''' représente un groupement alkyle ($C_1$-$C_6$) linéaire ou ramifié, éventuellement substitué par un groupement aryle ou hétéroaryle, pour conduire au composé de formule (XV) :

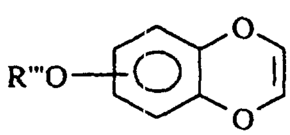

(XV)

dans laquelle R''' a la même définition que précédemment, l'ensemble des composés (XIIIa) et (XV) formant le composé de formule (XVI) :

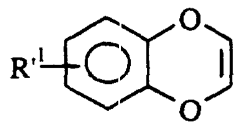

(XVI)

dans laquelle R'[1] représente un atome d'hydrogène, un groupement alkoxy ($C_1$-$C_6$) linéaire ou ramifié, éventuellement substitué par un groupement aryle ou hétéroaryle, sur lequel on condense, après l'action d'une base forte comme le butyl lithium par exemple, les électrophiles de formule (XVII):

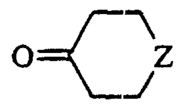

(XVII)

dans laquelle Z représente un groupement NCOOEt ou un groupement

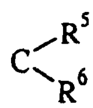

où $R^5$ et $R^6$ représentent simultanément un atome d'hydrogène ou forment ensemble un cycle

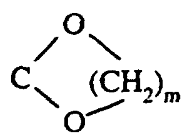

où m vaut 2 ou 3,
pour conduire au composé de formule (XVIII) :

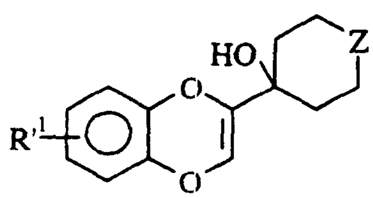

(XVIII)

dans laquelle R'[1] et Z ont la même définition que précédemment, qui est déshydraté en présence de chlorure de mésyle par exemple, pour obtenir le composé de formule (XIX) :

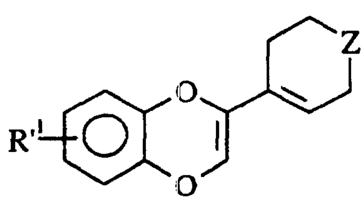

(XIX)

dans laquelle R'[1] et Z sont tels que définis précédemment, sur lequel on condense l'acétylène dicarboxylate

de méthyle ou le propiolate de méthyle pour obtenir le composé de formule (XX) :

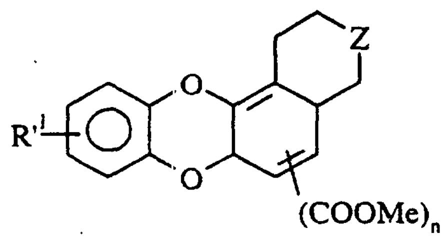

(XX)

dans laquelle $R'^1$ et Z ont la même signification que précédemment et n vaut 1 ou 2, que l'on soumet à l'action d'un agent oxydant comme l'orthochloranile ou la 2,3-dichloro-5,6-dicyano-1,4-benzoquinone pour conduire

- au composé de formule (I/n), cas particulier des composés de formule (I):

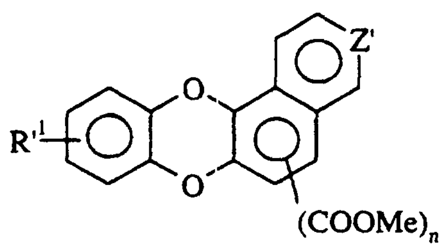

(I/n)

dans laquelle $R'^1$ et n ont la même définition que précédemment et Z' représente un atome d'azote ou un groupement C-$R^7$ où $R^7$ représente un atome d'hydrogène ou un groupement O-$(CH_2)_m$-OH dans lequel m vaut 2 ou 3,

- ou au composé de formule (XXI):

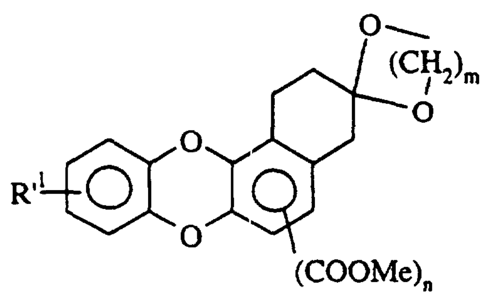

(XXI)

dans laquelle $R'^1$, n et m sont tels que définis précédemment, qui est successivement soumis au déblocage de l'acétal en milieu acide, à la formation d'un éther silylé, à l'oxydation par la 2,3-dichloro-5,6-dicyano-1,4-benzoquinone, et déblocage de l'éther silylé par un agent fluoré, pour conduire au composé de formule (I/o), cas particulier des composés de formule (I):

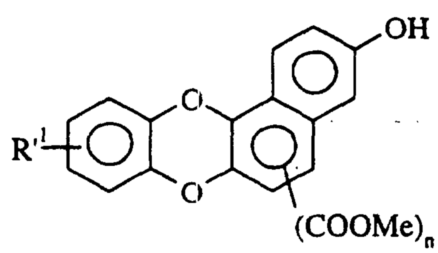

(I/o)

dans laquelle R'$^1$ et n ont la même définition que précédemment, sur lequel on condense divers électrophiles $R^8$-Halogène où $R^3$ représente un groupement alkyle ($C_1$-$C_6$) linéaire ou ramifié, éventuellement substitué par un groupement aryle ou hétéroaryle, pour obtenir les composés de formule (I/p), cas particulier des composés de formule (I):

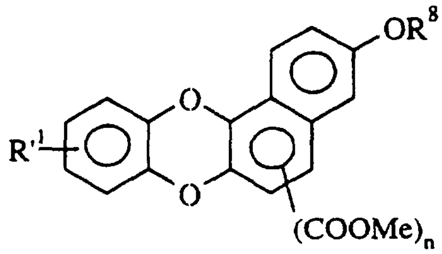

(I/p)

dans laquelle R'$^1$, n et $R^8$ sont tels que définis précédemment, les composés de formules (I/n), (I/o) et (I/p) formant l'ensemble des composés de formule (I/q), cas particulier des composés de formule (I) :

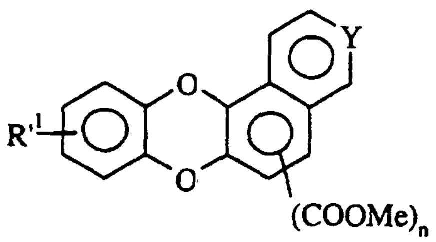

(I/q)

dans laquelle R'$^1$ et n sont tels que définis précédemment et Y a la même définition que dans la formule (I), composé (I/q) sur lequel :

* lorsque n vaut 1 ou 2, on condense les diamines de formule HNR"aR"b dans laquelle R"a représente un groupement dialkyle ($C_1$-$C_6$) -linéaire ou ramifié- aminoalkyle ($C_1$-$C_6$) linéaire ou ramifié (chaque partie alkyle étant identique ou différente indépendamment l'une de l'autre) ou un groupement alkyle ($C_1$-$C_6$), linéaire ou ramifié, substitué par au moins un groupement hydroxy, et R"b représente un atome d'hydrogène ou un groupement alkyle ($C_1$-$C_6$) linéaire ou ramifié pour conduire aux composés de formule (I/r), cas particulier des composés de formule (I):

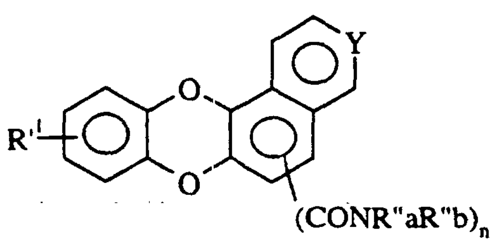

(I/r)

dans laquelle R'$^1$, R"a, R"b, n et Y ont la même définition que précédemment,

* ou, lorsque n vaut 1 ou 2, que l'on soumet à des conditions réductrices pour conduire à l'alcool correspondant qui subit une oxydation en aldéhyde en présence d'oxyde de manganèse pour conduire au composé de formule (XXII) :

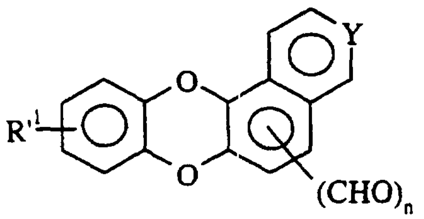

(XXII)

dans laquelle R'1, Y et n sont tels que définis précédemment, qui est condensé, en présence de chlorure de zinc et de cyanoborohydrure de sodium, avec les diamines de formule HNR"aR"b dans laquelle R"a représente un groupement dialkyle ($C_1$-$C_6$) -linéaire ou ramifié-aminoalkyle ($C_1$-$C_6$) linéaire ou ramifié ou un groupement alkyle ($C_3$-$C_6$) linéaire ou ramifié substitué par au moins un groupement hydroxy, et R"b représente un atome d'hydrogène ou un groupement alkyle ($C_1$-$C_6$) linéaire ou ramifié pour conduire aux composés de formule (I/s), cas particulier des composés de formule (I) :

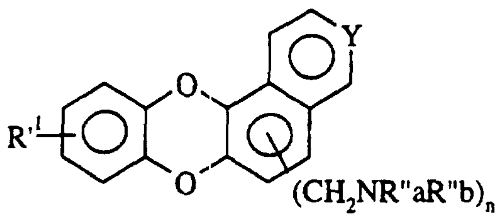

(I/s)

dans laquelle R'1, R"a, R"b, Y et n ont la même définition que précédemment,

* ou lorsque n vaut 2 aux composés de formule (I/t), cas particulier des composés de formule (I) :

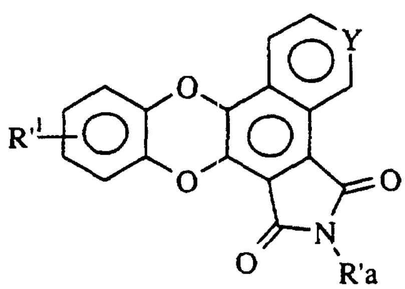

(I/t)

dans laquelle R'1, R'a et Y ont la même définition que précédemment,

les composés (I/a-t) formant l'ensemble des composés de l'invention que l'on purifie, le cas échéant, selon une technique classique de purification, qui peuvent, si on le désire, être séparés en leurs différents isomères optiques ou géométriques ou salifiés avec un acide ou une base pharmaceutiquement acceptable.

**9.** Compositions pharmaceutiques contenant comme principe actif au moins un composé selon l'une quelconque des revendications 1 à 7, seul ou en combinaison avec un ou plusieurs excipients ou véhicules inertes, non toxiques, pharmaceutiquement acceptables.

**10.** Compositions pharmaceutiques selon la revendication 9 contenant au moins un principe actif, agent antitumoral selon l'une quelconque des revendications 1 à 7, utiles dans le traitement des cancers.

**Patentansprüche**

**1.** Verbindungen der Formel (I):

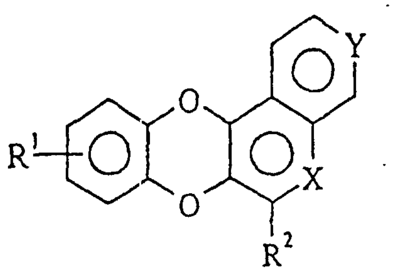

(I)

in der:

$R^1$ ein Wasserstoffatom oder eine Gruppe der Formel O-R darstellt, worin R ein Wasserstoffatom, eine geradkettige oder verzweigte, gegebenenfalls durch eine Aryl- oder Heteroarylgruppe substituierte ($C_1$-$C_6$)-Alkylgruppe bedeutet,

$R^2$ ein Wasserstoffatom, ein Halogenatom, eine Hydroxygruppe, eine geradkettige oder verzweigte ($C_1$-$C_6$)-Alkoxygruppe, eine Formylgruppe, eine Gruppe $CF_3SO_3$, eine Cyanogruppe, eine geradkettige oder verzweigte ($C_1$-$C_6$)-Alkoxycarbonylgruppe, eine Aryloxycarbonylgruppe, eine Gruppe NR'aR'b, in der R'a eine ($C_1$-$C_6$)-Dialkyl-aminoalkylgruppe (wobei jeder Alkylrest unabhängig von den anderen aus einer gleichartigen oder verschiedenen, geradkettigen oder verzweigten Kette, die 1 bis 6 Kohlenstoffatome enthält, gebildet ist) und R'b ein Wasserstoffatom oder eine geradkettige oder verzweigte ($C_1$-$C_6$)-Alkylgruppe darstellen, oder eine Gruppe BNR"aR"b, in der B eine Carbonylgruppe oder eine Methylengruppe bedeuten, R"a und R"b jeweils die gleichen Bedeutungen besitzen wie R'a und R'b oder R"a eine geradkettige oder verzweigte ($C_1$-$C_6$)-Alkylgruppe, die durch mindestens eine Hydroxygruppe substituiert ist, darstellt, bedeutet,

X ein Stickstoffatom (welches gegebenenfalls durch eine geradkettige oder verzweigte R'c ($C_1$-$C_6$)-Alkylgruppe substituiert ist) oder eine Gruppe C-$R^3$, worin $R^3$ ein Wasserstoffatom, eine geradkettige oder verzweigte ($C_1$-$C_6$)-Alkoxycarbonylgruppe oder eine Gruppe BNR"aR"b darstellt, worin B eine Carbonylgruppe oder eine Methylengruppe bedeutet und R"a und R"b die oben angegebenen Bedeutungen besitzen, bedeutet, oder $R^2$ und X, wenn X eine Gruppe C-$R^3$ darstellt, zusammen mit dem sie tragenden Kohlenstoffatom einen Ring der Formel (II) bilden:

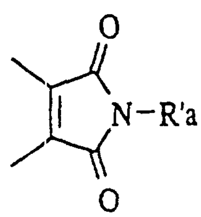

(II)

in der R'a die oben angegebenen Bedeutungen besitzt,

Y x ein Stickstoffatom oder eine Gruppe C-$R^4$ bedeutet, worin $R^4$ ein Wasserstoffatom oder eine Gruppe der Formel O-R" darstellt, worin R" ein Wasserstoffatom, eine geradkettige oder verzweigte (gegebenenfalls durch eine Hydroxygruppe substituierte) ($C_1$-$C_6$)-Alkylgruppe, eine Arylgruppe oder eine Heteroarylgruppe bedeutet,

mit der Maßgabe, daß, wenn X ein Stickstoffatom und Y eine Gruppe CH bedeuten, oder wenn X eine Gruppe CH und Y ein Stickstoffatom darstellen, oder, wenn X und Y gleichzeitig eine CH-Gruppe bedeuten, dann $R^1$ und $R^2$ nicht gleichzeitig Wasserstoffatome bedeuten können,
wobei es sich versteht, daß der Begriff Aryl für eine Phenylgruppe oder Naphthylgruppe steht, die gegebenenfalls in identischer oder unterschiedlicher Weise mit einem oder mehreren Halogenatomen oder geradkettigen oder verzweigten ($C_1$-$C_6$)-Alkylgruppen, Hydroxygruppen, geradkettigen oder verzweigten ($C_1$-$C_6$)-Alkoxygruppen oder Trihalogenmethylgruppen substituiert sind,
und der Begriff Heteroaryl für eine aromatische mono- oder bicyclische Gruppe steht, die 1 oder 2 Heteroatome ausgewählt aus O, S und N enthält und gegebenenfalls in identischer oder unterschiedlicher Weise substituiert ist durch ein oder mehrere Halogenatome oder geradkettige oder verzweigte ($C_1$-$C_6$)-Alkylgruppen, Hydroxygrup-

pen, geradkettige oder verzweigte ($C_1$-$C_6$)-Alkoxygruppen oder Trihalogenmethylgruppen substituiert ist, deren Isomere sowie deren Additionssalze mit einer pharmazeutisch annehmbaren Säure oder Base.

**2.** Verbindungen der Formel (I) nach Anspruch 1, worin $R^2$ eine Gruppe NR'aR'b bedeutet, worin R'a eine (geradkettig oder verzweigt $C_1$-$C_6$)-Dialkyl-amino(geradkettig oder verzweigt $C_1$-$C_6$)-alkylgruppe, wobei jeder Alkylrest unabhängig von den anderen identisch oder verschieden ist, und R'b ein Wasserstoffatom oder eine geradkettige oder verzweigte ($C_1$-$C_6$)-Alkylgruppe bedeuten, deren Isomere sowie deren Additionssalze mit einer pharmazeutisch annehmbaren Säure oder Base.

**3.** Verbindungen der Formel (I) nach Anspruch 1, worin $R^2$ eine Gruppe BNR"aR"b darstellt, worin B eine Carbonylgruppe oder eine Methylengruppe, R"a eine (geradkettig oder verzweigt $C_1$-$C_6$)-Dialkyl-amino-(geradkettig oder verzweigt $C_1$-$C_6$)-alkylgruppe, worin jeder Alkylrest unabhängig von den anderen identisch oder verschieden ist, und R"b ein Wasserstoffatom oder eine geradkettige oder verzweigte ($C_1$-$C_6$)-Alkylgruppe bedeuten, deren Isomere sowie deren Additionssalze mit einer pharmazeutisch annehmbaren Säure oder Base.

**4.** Verbindungen der Formel (I) nach Anspruch 1, worin X eine Gruppe C-$R^3$ darstellt, worin $R^3$ eine Gruppe BNR"aR"b darstellt, worin B eine Carbonylgruppe oder eine Methylengruppe, R"a eine (geradkettig oder verzweigt $C_1$-$C_6$)-Dialkylamino-(geradkettig oder verzweigt $C_1$-$C_6$)-alkylgruppe, wobei jeder Alkylrest unabhängig von den anderen identisch oder verschieden ist, und R"b ein Wasserstoffatom oder eine geradkettige oder verzweigte ($C_1$-$C_6$)-Alkylgruppe bedeuten, deren Isomere sowie deren Additionssalze mit einer pharmazeutisch annehmbaren Säure oder Base.

**5.** Verbindungen der Formel (I) nach Anspruch 1, worin $R^2$ und X, wenn X eine Gruppe C-$R^3$ darstellt, zusammen mit dem sie tragenden Kohlenstoffatom einen Ring der Formel (II) bilden:

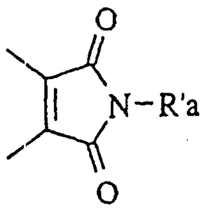

(II)

in der R'a eine (geradkettig oder verzweigt $C_1$-$C_6$)-Dialkyl-amino-(geradkettig oder verzweigt $C_1$-$C_6$)-alkylgruppe darstellt, wobei jeder Alkylrest unabhängig von den anderen identisch oder verschieden ist, deren Isomere sowie deren Additionssalze mit einer pharmazeutisch annehmbaren Säure oder Base.

**6.** Verbindungen der Formel (I) nach Anspruch 5, worin R'a eine Dimethylaminoethylgruppe bedeutet, deren Isomere sowie deren Additionssalze mit einer pharmazeutisch annehmbaren Säure oder Base.

**7.** Verbindung nach Anspruch 1, nämlich N 2-(7,12-Dioxa-3-hydroxy-benzo[a]-anthracen-5,6-maleimido)-N1,N1-dimethylethylendiamin-Hydrochlorid und dessen Additionssalze mit einer pharmazeutisch annehmbaren Säure oder Base.

**8.** Verfahren zur Herstellung der Verbindungen der Formel (I), **dadurch gekennzeichnet, daß** man als Ausgangsprodukt:

- *für die Verbindungen, worin X ein Stickstoffatom oder die Gruppe CH darstellt,* die Verbindung der Formel (III) verwendet:

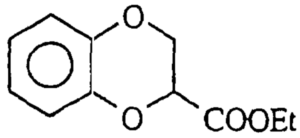

(III)

welche - entweder dibromiert und dann mit m-Chlorperbenzoesäure behandelt und dann der Einwirkung von Kaliumiodid unterworfen wird zur Bildung der Verbindung (IIIa):

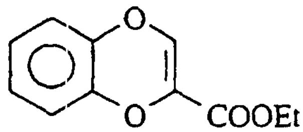

(IIIa)

- oder mit Hilfe einer Friedel-Crafts-Reaktion acyliert, dibromiert und dann mit m-Chlorperbenzoesäure behandelt wird zur Bildung der Verbindung der Formel (IV):

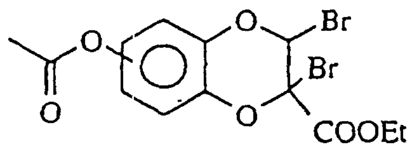

(IV)

welche der Einwirkung von Natriurniodid und dann einer Base, wie Natriumethylat, unterworfen wird zur Bildung der Verbindung (V):

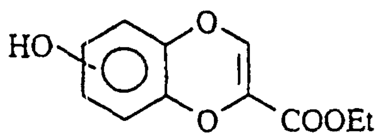

(V)

welche man mit elektrophilen Verbindungen der Formel R'''-Halogen, worin R''' eine geradkettige oder verzweigte, gegebenenfalls mit einer Arylgruppe oder einer Heteroarylgruppe substituierte ($C_1$-$C_6$)-Alkylgruppe darstellt, kondensiert zur Bildung der Verbindung der Formel (VI):

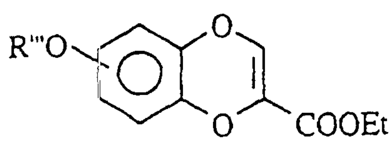

(VI)

in der R''' die oben angegebenen Bedeutungen besitzt,
wobei die Gesamtheit der Verbindungen (IIIa) und (VI) die Verbindungen der Formel (VII) bildet:

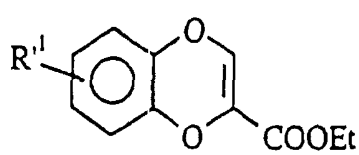

(VII)

in der $R'^1$ ein Wasserstoffatom, eine geradkettige oder verzweigte, gegebenenfalls mit einer Aryl- oder einer Heteroarylgruppe substituierte ($C_1$-$C_6$)-Alkoxygruppe bedeutet,
welche man nach der Verseifung mit beispielsweise wäßrigem Natriumhydroxid mit Diethylamin in Gegenwart eines Kupplungsmittels, wie 1,3-Dimethylaminopropyl-3-ethylcarbodiimid, kondensiert zur Bildung der Verbindung der Formel (VIII):

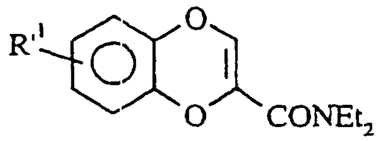

(VIII)

in der R'1 die oben angegebenen Bedeutungen besitzt, welche der Einwirkung einer starken Base und dann von Trimethylzinnchlorid unterworfen wird zur Bildung der Verbindung der Formel (IX):

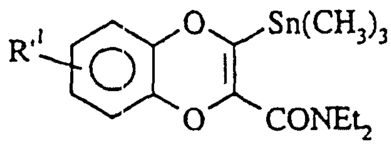

(IX)

in der R'1 die oben angegebenen Bedeutungen besitzt, welche man in Gegenwart von Kupferiodid und eines Komplexes eines Übergangsmetalls, wie Palladium:

- **entweder** mit N-tert.-Butoxycarbonyl-2-iodanilin kondensiert zur Bildung der Verbindung der Formel (X):

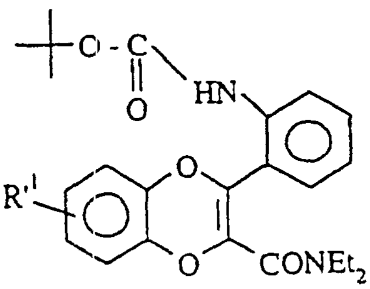

(X)

in der R'1 die oben angegebenen Bedeutungen besitzt, welche nach der sauren Hydrolyse die Verbindung der Formel (XI) liefert:

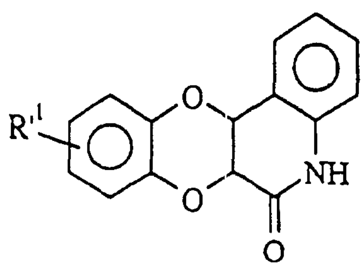

(XI)

in der R'1 die oben angegebenen Bedeutungen besitzt, welche man mit $POCl_3$ behandelt zur Bildung der Verbindung der Formel (I/a), einem Sonderfall der Verbindungen der Formel (I):

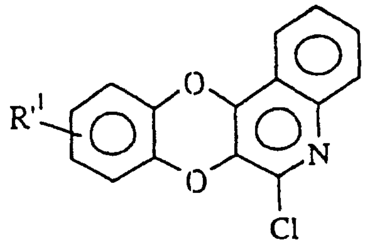

(I/a)

in der $R'^1$ die oben angegebenen Bedeutungen besitzt,
welche man dann, wenn $R'^1$ eine geradkettige oder verzweigte $(C_1-C_6)$-Alkoxygruppe darstellt, mit einem Dealkylierungsmittel, wie beispielsweise Bortribromid, behandelt zur Bildung der Verbindung der Formel (I/b), einem Sonderfall der Verbindungen der Formel (I):

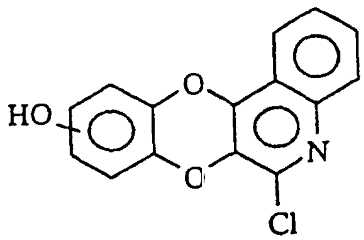

(I/b)

wobei die Gesamtheit der Verbindungen der Formeln (I/a) und (I/b) die Verbindung der Formel (I/c) bildet, einem Sonderfall der Verbindungen der Formel (I):

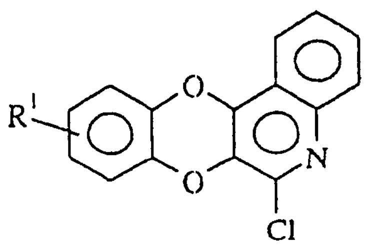

(I/c)

in der $R^1$ die bezüglich der Formel (I) angegebenen Bedeutungen besitzt, welche Verbindung der Formel (I/c):

* man der Einwirkung eines Reduktionsmittels, wie Zink, unterwirft zur Bildung der Verbindung der Formel (I/d), einem Sonderfall der Verbindungen der Formel (I):

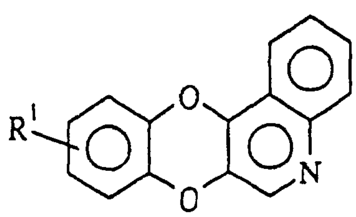

(I/d)

in der $R^1$ die oben angegebenen Bedeutungen besitzt,
welche man einer N-Alkylierung mit einem Alkylierungsmittel, wie Methyliodid, unterwirft zur Bildung der Verbindung der Formel (I/e), einem Sonderfall der Verbindungen der Formel (I):

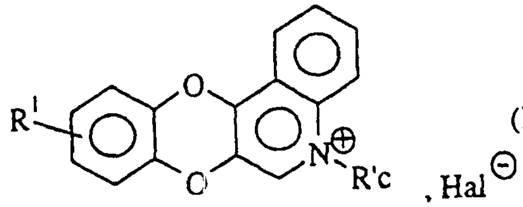

(I/e)

in der R'c eine geradkettige oder verzweigte ($C_1$-$C_6$)-Alkylgruppe und Hal ein Halogen bedeuten,

* **oder** welche man mit Diaminen der Formel HNR'aR'b, worin R'a eine (geradkettig oder verzweigt $C_1$-$C_6$)-Dialkyl-amlno-(geradkettig oder verzweigt $C_1$-$C_6$)-alkylgruppe, wobei jeder Alkylrest unabhängig von den anderen identisch oder verschieden ist, und R'b ein Wasserstoffatom oder eine geradkettige oder verzweigte ($C_1$-$C_6$)-Alkylgruppe bedeuten, kondensiert zur Bildung der Verbindung der Formel (I/f), einem Sonderfall der Verbindungen der Formel (I):

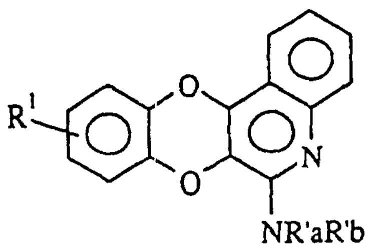

(I/f)

in der $R^1$, R'a und R'b die oben angegebenen Bedeutungen besitzen,

- oder mit 2-Iodtoluol kondensiert zur Bildung der Verbindung der Formel (XII):

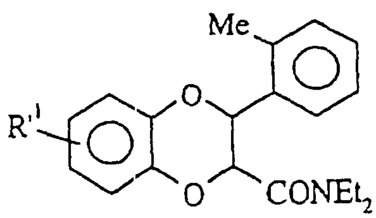

(XII)

in der $R'^1$ die oben angegebenen Bedeutungen besitzt,
welche nach der Einwirkung einer starken Base, wie Lithiumdiisopropylamid, zu der Verbindung der Formel (I/g), einem Sonderfall der Verbindungen der Formel (I) führt:

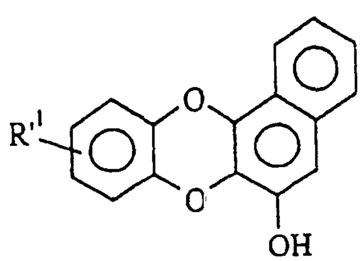

(I/g)

in der $R'^1$ die oben angegebenen Bedeutungen besitzt,
welche Verbindung der Formel (I/g) man:

* in dem Fall, da $R'^1$ eine geradkettige oder verzweigte ($C_1$-$C_6$)-Alkoxygruppe darstellt, der Einwirkung

eines Dealkylierungsmittels, wie beispielsweise Bortribromid, unterwirft zur Bildung der Verbindung der Formel (I/h), einem Sonderfall der Verbindungen der Formel (I):

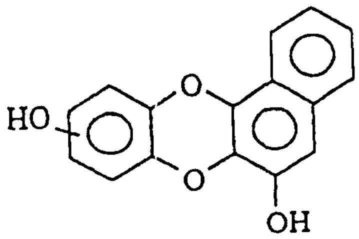

(I/h)

* oder man der aufeinanderfolgenden Einwirkung von Trifluormethansulfonsäureanhydrid, eines Cyanids in Gegenwart eines Komplexes eines Übergangsmetalls, wie Palladium, und eines Reduktionsmittels, wie Diisopropylaluminiumhydrid, unterwirft zur Bildung der Verbindung der Formel (I/i), einem Sonderfall der Verbindungen der Formel (I):

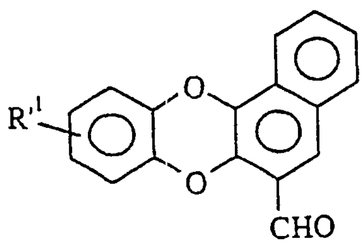

(I/i)

in der $R'^1$ die oben angegebenen Bedeutungen besitzt, welche durch Einwirkung eines Oxidationsmittels, wie Manganoxid in Gegenwart von Methanol, zu dem Methylester oxidiert wird, so daß man die Verbindung der Formel (I/j), einem Sonderfall der Verbindungen der Formel (I) erhält:

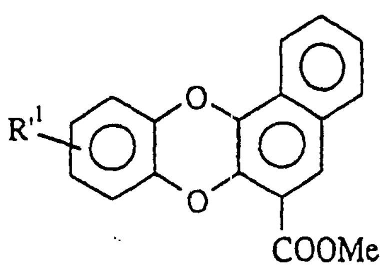

(I/j)

in der $R'^1$ die oben angegebenen Bedeutungen besitzt, welche man dann, wenn $R'^1$ eine geradkettige oder verzweigte ($C_1$-$C_6$)-Alkoxygruppe darstellt, mit einem Dealkylierungsmittel, wie Bortribromid, behandelt zur Bildung der Verbindung der Formel (I/k), einem Sonderfall der Verbindungen der Formel (I):

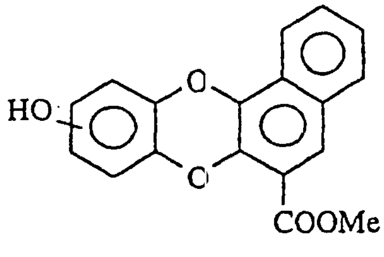

(I/k)

wobei die Gesamtheit der Verbindungen (I/j) und (I/k) die Verbindungen der Formel (I/1) bildet, einem Sonderfall der Verbindungen der Formel (I):

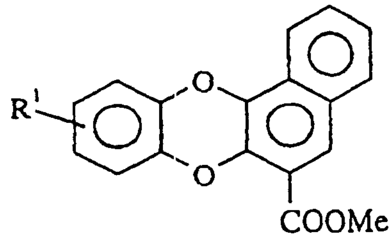

(I/l)

in der $R^1$ die bezüglich der Formel (I) angegebenen Bedeutungen besitzt, welche man mit Diaminen der Formel HNR'aR'b, worin R'a eine (geradkettig oder verzweigt $C_1$-$C_6$)-Dialkyl-amino-(geradkettig oder verzweigt $C_1$-$C_6$)-alkylgruppe (wobei jeder Alkylrest identisch oder verschieden ist) und R'b ein Wasserstoffatom oder eine geradkettige oder verzweigte ($C_1$-$C_6$)-Alkylgruppe bedeuten, kondensiert zur Bildung der Verbindung der Formel (I/m), einem Sonderfall der Verbindungen der Formel (I):

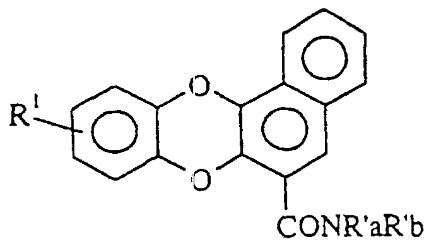

(I/m)

worin $R^1$, R'a und R'b die oben angegebenen Bedeutungen besitzen, wobei es sich versteht, daß die Verbindungen (I/m) ausgehend von der Verbindung (I/k), welche der Einwirkung von elektrophilen Verbindungen der Formel $R'^1$-Halogen, worin $R'^1$ die oben angegebenen Bedeutungen besitzt, in alkalischem Medium erhalten werden können,

- *für die Verbindungen, worin X kein Stickstoffatom darstellt*, die Verbindung der Formel (XIII) verwendet:

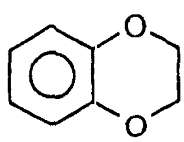

(XIII)

welche:

- entweder dibromiert und dann mit m-Chlorperbenzoesäure behandelt und der Einwirkung von Kaliumiodid unterworfen wird zur Bildung der Verbindung (XIIIa):

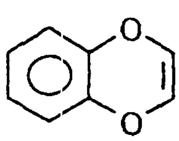

(XIIIa)

- oder gemäß einer Friedel-Crafts-Reaktion acyliert, dibromiert, mit m-Chlorperbenzoesäure behandelt, der Einwirkung von Natriumiodid und dann einer Base unterworfen wird zur Bildung der Verbindung (XIV):

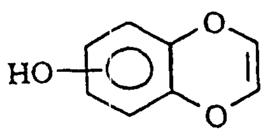

(XIV)

welche man mit elektrophilen Verbindungen der Formel R'''-Halogen. worin R''' eine geradkettige oder verzweigte, gegebenenfalls mit einer Aryl- oder Heteroarylgruppe substituierte ($C_1$-$C_6$)-Alkylgruppe darstellt, kondensiert zur Bildung der Verbindung der Formel (XV):

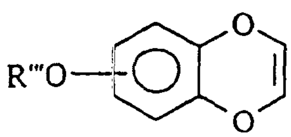

(XV)

in der R''' die oben angegebenen Bedeutungen besitzt, wobei die Gesamtheit der Verbindungen (XIIIa) und (XV) die Verbindung der Formel (XVI) bildet:

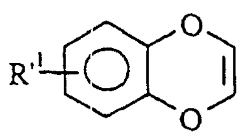

(XVI)

in der $R'^1$ ein Wasserstoffatom, eine geradkettige oder verzweigte, gegebenenfalls durch eine Aryl- oder Heteroarylgruppe substituierte ($C_1$-$C_6$)-Alkoxygruppe darstellt, welche man nach der Einwirkung einer starken Base, wie beispielsweise Butyllithium, mit elektrophilen Verbindungen der Formel (XVII) kondensiert:

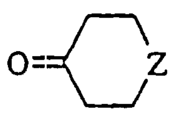

(XVII)

in der Z eine Gruppe NCOOEt oder eine Gruppe

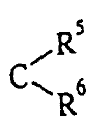

darstellt, worin $R^5$ und $R^6$ gleichzeitig ein Wasserstoffatom bilden oder gemeinsam den Ring

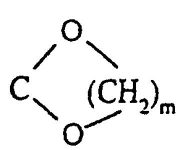

bilden, in dem m 2 oder 3 bedeutet,
zur Bildung der Verbindung der Formel (XVIII):

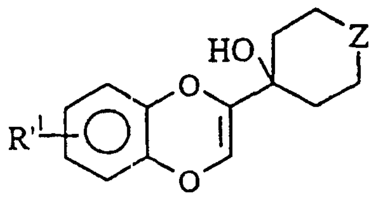

(XVIII)

in der $R'^1$ und Z die oben angegebenen Bedeutungen besitzen, welche beispielsweise in Gegenwart von Mesylchlorid dehydratisiert wird zur Bildung der Verbindung der Formel (XIX):

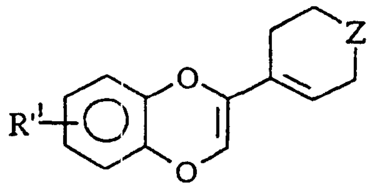

(XIX)

in der $R'^1$ und Z die oben angegebenen Bedeutungen besitzen, welche man mit Acetylendicarbonsäuremethylester oder Propiolsäuremethylester behandelt zur Bildung der Verbindung der Formel (XX):

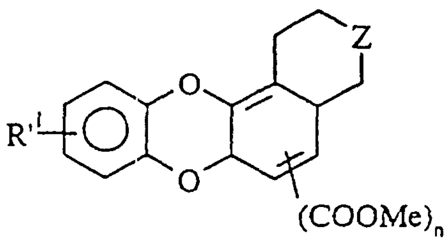

(XX)

in der $R'^1$ und Z die oben angegebenen Bedeutungen besitzen und n 1 oder 2 darstellt, welche man der Einwirkung eines Oxidationsmittels, wie o-Chloranil oder 2,3-Dichlor-5,6-dicyano-1,4-benzochinon, unterwirft, so daß man

- die Verbindung der Formel (I/n), einem Sonderfall der Verbindungen der Formel (I) erhält:

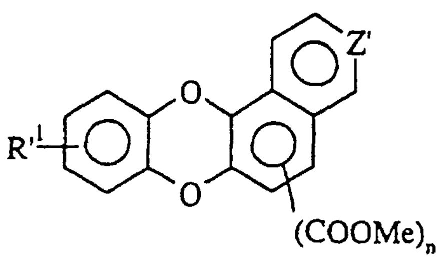

(I/n)

in der $R'^1$ und n die oben angegebenen Bedeutungen besitzen und Z' ein Stickstoffatom oder eine Gruppe C-$R^7$ darstellt, worin $R^7$ ein Wasserstoffatom oder eine Gruppe O-$CH_2)_m$-OH, in der m den Wert 2 oder 3 besitzt, bedeutet,

- oder die Verbindung der Formel (XXI) erhält:

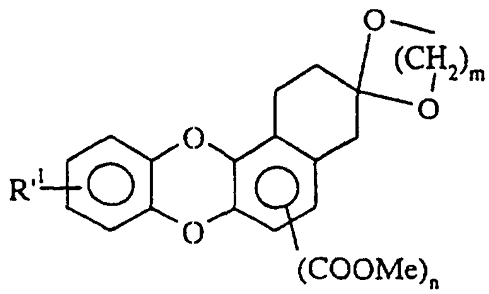

(XXI)

in der R'[1], n und m die oben angegebenen Bedeutungen besitzen, welche nacheinander der Acetalspaltung in saurem Medium, der Bildung eines Silylethers, der Oxidation mit 2,3-Dichlor-5,6-dicyano-1,4-benzochinon und der Abspaltung des Silylethers mit einem Fluor-haltigen Mittel unterworfen wird zur Bildung der Verbindungen der Formel (I/o), einem Sonderfall der Verbindungen der Formel (I):

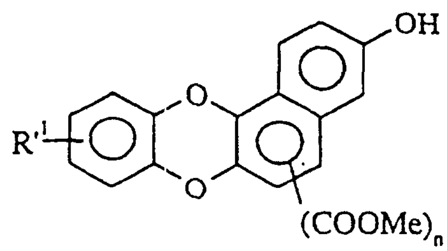

(I/o)

in der R'[1] und n die oben angegebenen Bedeutungen besitzen, welche man mit verschiedenen elektrophilen Verbindungen $R^8$-Halogen, worin $R^8$ eine geradkettige oder verzweigte, gegebenenfalls mit einer Aryl- oder Heteroarylgruppe substituierte ($C_1$-$C_6$)-Alkylgruppe darstellt, kondensiert zur Bildung der Verbindungen der Formel (I/p), einem Sonderfall der Verbindungen der Formel (I):

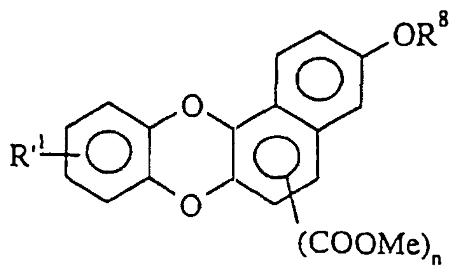

(I/p)

in der R'[1], n und $R^8$ die oben angegebenen Bedeutungen besitzen, welche Verbindungen der Formeln (I/n), (I/o) und (I/p) die Gesamtheit der Verbindungen der Formel (I/q) bilden, einem Sonderfall der Verbindungen der Formel (I):

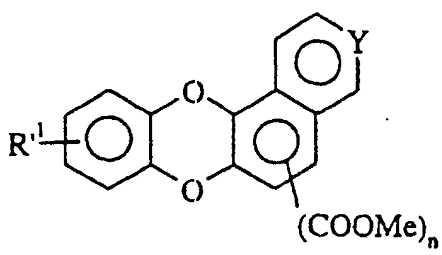

(I/q)

in der R'1 und n die oben angegebenen Bedeutungen besitzen und Y die bezüglich der Formel (I) angegebebenen Bedeutungen besitzt, welche Verbindung (I/q):

* dann, wenn n 1 oder 2 bedeutet, mit Diaminen der Formel HNR"aR"b kondensiert wird, in der R"a eine (geradkettig oder verzweigt $C_1$-$C_6$)-Dialkyl-amino(geradkettig oder verzweigt $C_1$-$C_6$)-alkylgruppe (wobei jeder Alkylrest unabhängig von den anderen identisch oder verschieden ist) oder eine geradkettige oder verzweigte, mindestens mit einer Hydroxygruppe substituierte ($C_1$-$C_6$)-Alkylgruppe und R"b ein Wasserstoffatom oder eine geradkettige oder verzweigte ($C_1$-$C_6$)-Alkylgruppe bedeuten, zur Bildung der Verbindungen der Formel (I/r), einem Sonderfall der Verbindungen der Formel (I):

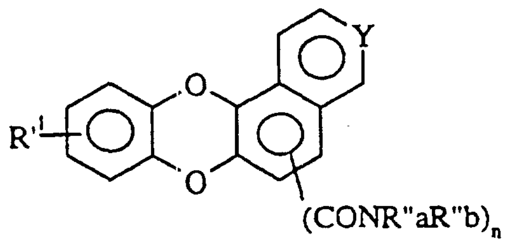

(I/r)

in der R'1, R"a, R"b, n und Y die oben angegebenen Bedeutungen besitzen,

* oder dann, wenn n 1 oder 2 bedeutet, reduzierenden Bedingungen unterworfen wird zur Bildung des entsprechenden Alkohols, welcher in Gegenwart von Manganoxid einer Oxidation zu dem Aldehyd unterworfen wird zur Bildung der Verbindung der Formel (XXII):

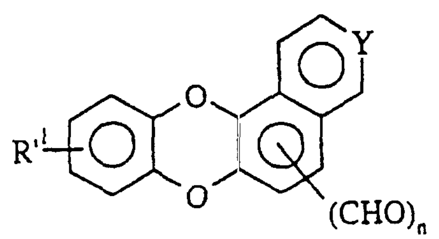

(XXII)

in der R'1, Y und n die oben angegebenen Bedeutungen besitzen,
welche in Gegenwart von Zinkchlorid und Natriumcyanborhydrid mit Diaminen der Formel HNR"aR"b kondensiert wird, in der R"a eine (geradkettig oder verzweigt $C_1$-$C_6$)-Dialkyl-amino-(geradkettig oder verzweigt $C_1$-$C_6$)-alkylgruppe oder eine geradkettige oder verzweigte, mit mindestens einer Hydroxygruppe substituierte ($C_1$-$C_6$)-Alkylgruppe und R"b ein Wasserstoffatom oder eine geradkettige oder verzweigte ($C_1$-$C_6$)-Alkylgruppe bedeuten, zur Bildung der Verbindungen der Formel (I/s), einem Sonderfall der Verbindungen der Formel (I):

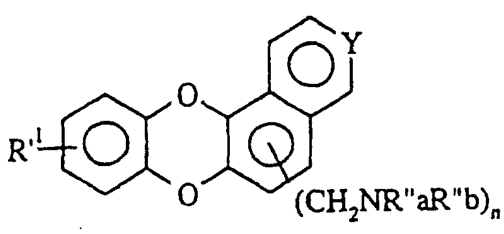

(I/s)

in der R'1, R"a, R"b, Y und n die oben angegebenen Bedeutungen besitzen,

* oder, wenn n 2 bedeutet, mit Verbindungen der Formel (I/t), einem Sonderfall der Verbindungen der Formel (I) kondensiert wird:

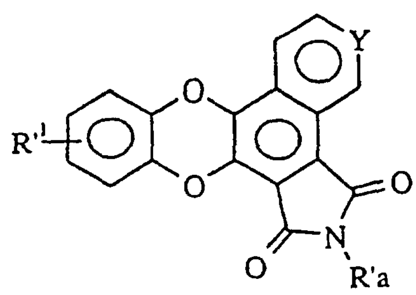

(I/t)

in der $R'^1$, R'a und Y die oben angegebenen Bedeutungen besitzen,
wobei die Verbindungen (I/a-t) die Gesamtheit der erfindungsgemäßen Verbindungen bilden, welche man gegebenenfalls mit Hilfe einer klassischen Reinigungsmethode reinigt, welche gewünschtenfalls in ihre verschiedenen optischen oder geometrischen Isomeren aufgetrennt werden können oder mit Hilfe einer pharmazeutisch annehmbaren Säure oder Base in ihre Salze überführt werden können.

**9.** Pharmazeutische Zubereitungen enthaltend als Wirkstoff mindestens eine Verbindung nach einem der Ansprüche 1 bis 7 allein oder in Kombination mit einem oder mehreren inerten, nichttoxischen, pharmazeutisch annehmbaren Trägermaterialien.

**10.** Pharmazeutische Zubereitungen nach Anspruch 9 enthaltend mindestens einen antitumoral wirksamen Wirkstoff nach einem der Ansprüche 1 bis 7 zur Behandlung von Krebserkrankungen.

**Claims**

**1.** Compounds of formula (I):

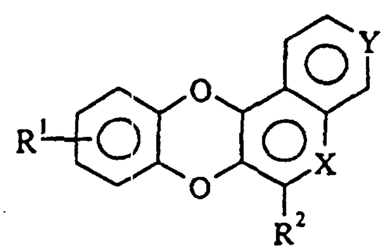

(I)

wherein:

$R^1$ represents a hydrogen atom or a group of formula O-R, wherein R represents a hydrogen atom, or a linear or branched $(C_1\text{-}C_6)$alkyl group, optionally substituted by an aryl or heteroaryl group,

$R^2$ represents a hydrogen atom, a halogen atom, a hydroxy group, a linear or branched $(C_1\text{-}C_6)$alkoxy group, a formyl group, a $CF_3SO_3$ group, a cyano group, a linear or branched $(C_1\text{-}C_6)$alkoxycarbonyl group, an aryloxycarbonyl group, an NR'aR'b group wherein R'a represents a dialkylaminoalkyl group $(C_1\text{-}C_6)$ (each alkyl moiety being made up of a linear or branched chain containing from 1 to 6 carbon atoms, and each being identical or different independently of the others), and R'b represents a hydrogen atom or a linear or branched $(C_1\text{-}C_6)$alkyl group, or a BNR"aR"b group wherein B represents a carbonyl or methylene group, R"a and R"b have the same meanings as R'a and R'b, respectively, or R"a represents a linear or branched $(C_1\text{-}C_6)$alkyl group substituted by at least one hydroxy group,

X represents a nitrogen atom (optionally substituted by a linear or branched $(C_1\text{-}C_6)$alkyl group R'c), or a C-$R^3$ group wherein $R^3$ represents a hydrogen atom, a linear or branched $(C_1\text{-}C_6)$alkoxycarbonyl group, or a BNR"aR"b group wherein B represents a carbonyl group or a methylene group and R"a and R"b are as defined

hereinbefore,
or $R^2$ and X, when X represents a C-$R^3$ group, with the carbon atom carrying them together form a ring of formula (II):

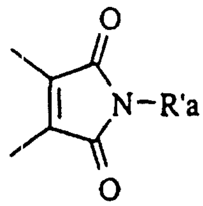

(II)

wherein R'a is as defined hereinbefore,

Y represents a nitrogen atom or a C-$R^4$ group wherein $R^4$ represents a hydrogen atom, or a group of formula O-R" wherein R" represents a hydrogen atom, a linear or branched ($C_1$-$C_6$)alkyl group (optionally substituted by a hydroxy group), or an aryl or heteroaryl group,

with the proviso that when X represents a nitrogen atom and Y represents a CH group, or when X represents a CH group and Y represents a nitrogen atom, or when X and Y simultaneously represent a CH group, then $R^1$ and $R^2$ cannot simultaneously represent a hydrogen atom,
it being understood that the term "aryl" denotes a phenyl or naphthyl group optionally substituted in identical or different manner by one or more halogen atoms or linear or branched ($C_1$-$C_6$)alkyl groups, hydroxy groups, linear or branched ($C_1$-$C_6$)alkoxy groups or trihalomethyl groups,
and the term "heteroaryl" denotes a mono- or bi-cyclic aromatic group containing 1 or 2 hetero atoms selected from O, S and N, optionally substituted in identical or different manner by one or more halogen atoms or linear or branched ($C_1$-$C_6$)alkyl groups, hydroxy groups, linear or branched ($C_1$-$C_6$)alkoxy groups or trihalomethyl groups,
their isomers and addition salts thereof with a pharmaceutically acceptable acid or base.

**2.** Compounds of formula (I) according to claim 1 wherein $R^2$ represents an NR'aR'b group wherein R'a represents a di-($C_1$-$C_6$)alkyl-($C_1$-$C_6$)aminoalkyl group, each alkyl moiety being linear or branched and identical or different independently of the others, and R'b represents a hydrogen atom or a linear or branched ($C_1$-$C_6$)alkyl group, their isomers and addition salts thereof with a pharmaceutically acceptable acid or base.

**3.** Compounds of formula (I) according to claim 1 wherein $R^2$ represents a BNR"aR"b group wherein B represents a carbonyl group or a methylene group, R"a represents a di-($C_1$-$C_6$)alkyl-($C_1$-$C_6$)aminoalkyl group, each alkyl moiety being linear or branched and identical or different independently of the others, and R"b represents a hydrogen atom or a linear or branched ($C_1$-$C_6$)alkyl group, their isomers and addition salts thereof with a pharmaceutically acceptable acid or base.

**4.** Compounds of formula (I) according to claim 1 wherein X represents a C-$R^3$ group wherein $R^3$ represents a BNR"aR"b group wherein B represents a carbonyl group or a methylene group, R"a represents a di-($C_1$-$C_6$)alkyl-($C_1$-$C_6$)aminoalkyl group, each alkyl moiety being linear or branched and identical or different independently of the others, and R"b represents a hydrogen atom or a linear or branched ($C_1$-$C_6$)alkyl group, their isomers and addition salts thereof with a pharmaceutically acceptable acid or base.

**5.** Compounds of formula (I) according to claim 1 wherein $R^2$ and X, when X represents a C-$R^3$ group, form together with the carbon atom carrying them a ring of formula (II):

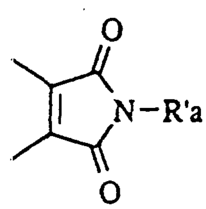

(II)

wherein R'a represents a di-($C_1$-$C_6$)alkyl-($C_1$-$C_6$)aminoalkyl group, each alkyl moiety being linear or branched and identical or different independently of the others, their isomers and addition salts thereof with a pharmaceutically acceptable acid or base.

**6.** Compounds of formula (I) according to claim 5 wherein R'a represents a dimethylaminoethyl group, their isomers and addition salts thereof with a pharmaceutically acceptable acid or base.

**7.** Compound according to claim 1 which is N2-(7,12-dioxa-3-hydroxy-benzo[a]anthracene-5,6-maleimido)-N1,N1-dimethylethylenediamine hydrochloride and its addition salts with a pharmaceutically acceptable base or acid.

**8.** Process for the preparation of compounds of formula (I), **characterised in that** there is used as starting material:

- *for compounds wherein X represents a nitrogen atom or a CH group, the compound of* formula (III):

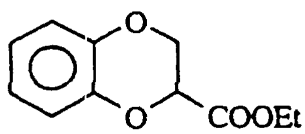

(III)

which is

- either dibrominated and then treated with metachloroperbenzoic acid and subjected to the action of potassium iodide to obtain the compound (IIIa):

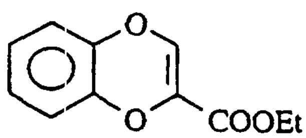

(IIIa)

- or acylated according to a Friedel Crafts reaction, dibrominated and then treated with metachloroperbenzoic acid, to yield the compound of formula (IV):

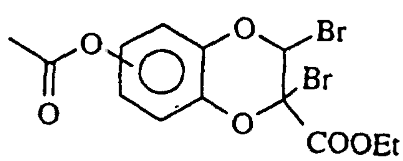

(IV)

which, when subjected to the action of sodium iodide and then of a base, such as sodium ethanolate, enables the compound (V) to be obtained:

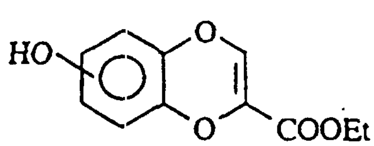

(V)

which is condensed with electrophiles of formula R'''-halogen, wherein R''' represents a linear or branched ($C_1$-$C_6$)alkyl group, optionally substituted by an aryl or heteroaryl group, to yield the compound of formula (VI):

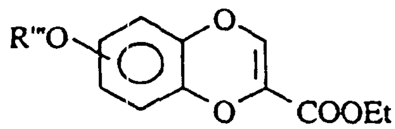

(VI)

wherein R''' is as defined hereinbefore,
the totality of the compounds (IIIa) and (VI) constituting the compound of formula (VII):

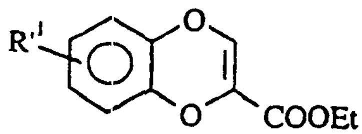

(VII)

wherein $R'^1$ represents a hydrogen atom, a linear or branched ($C_1$-$C_6$)alkoxy group, optionally substituted by an aryl or heteroaryl group,
which is condensed, after hydrolysis with aqueous sodium hydroxide for example, with diethylamine in the presence of a coupling agent, such as 1,3-dimethylaminopropyl-3-ethylcarbodiimide, to yield the compound of formula (VIII):

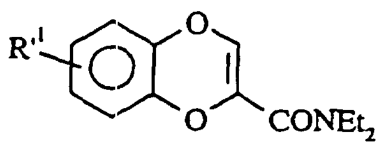

(VIII)

wherein $R'^1$ is as defined hereinbefore,
which is subjected to the action of a strong base, and then to trimethyltin chloride, to yield the compound of formula (IX):

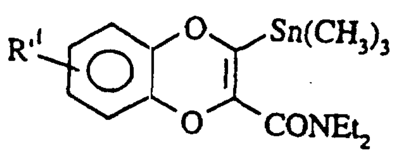

(IX)

wherein $R'^1$ is as defined hereinbefore,
which is condensed, in the presence of copper(I) iodide and a complex of a transition metal such as palladium:

- **either** with N-tert-butoxycarbonyl-2-iodoaniline, to obtain the compound of formula (X):

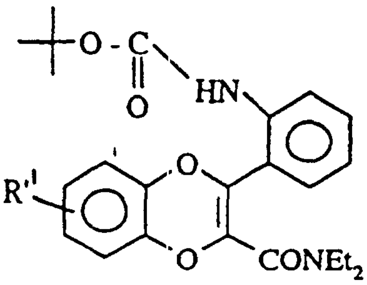

(X)

wherein $R'^1$ is as defined hereinbefore,
which, after acid hydrolysis, yields the compound of formula (XI):

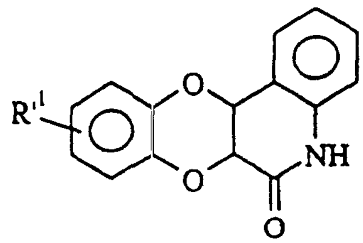

(XI)

wherein $R'^1$ is as defined hereinbefore,
which is reacted with $POCl_3$, to obtain the compound of formula (I/a), a particular case of the compounds of formula (I):

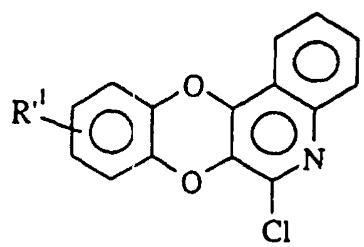

(I/a)

wherein $R'^1$ is as defined hereinbefore,
which, when $R'^1$ represents a linear or branched $(C_1\text{-}C_6)$alkoxy group, is subjected to the action of a dealkylating agent, such as boron tribromide for example, to yield the compound of formula (I/b), a particular case of the compounds of formula (I):

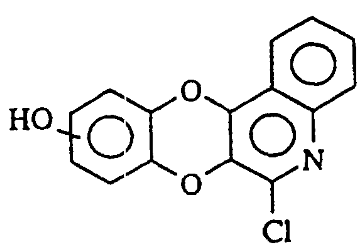

(I/b)

the totality of the compounds (I/a) and (I/b) constituting the compound of formula (I/c), a particular case of the compounds of formula (I):

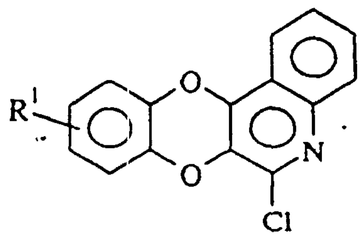

(I/c)

wherein $R^1$ is as defined for formula (I),
which compound of formula (I/c):

* is subjected to the action of a reducing agent, such as zinc, to yield the compound of formula (I/d), a particular case of the compounds of formula (I):

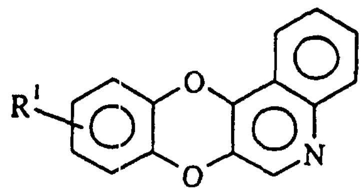

(I/d)

wherein $R^1$ is as defined hereinbefore,
which is subjected to N-alkylation by the action of an alkylating agent, such as methyl iodide, to yield the compound of formula (I/e), a particular case of the compounds of formula (I):

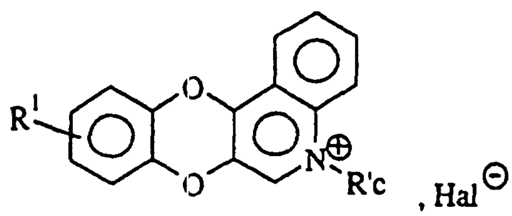

(I/e)

wherein R'c represents a linear or branched ($C_1$-$C_6$)alkyl group and Hal represents halogen,

* or is condensed with diamines of formula HNR'aR'b wherein R'a represents a di-($C_1$-$C_6$)alkyl-($C_1$-$C_6$) aminoalkyl group, each alkyl moiety being linear or branched and identical or different independently of the others, and R'b represents a hydrogen atom or a linear or branched ($C_1$-$C_6$)alkyl group, to yield the compound of formula (I/f), a particular case of the compounds of formula (I):

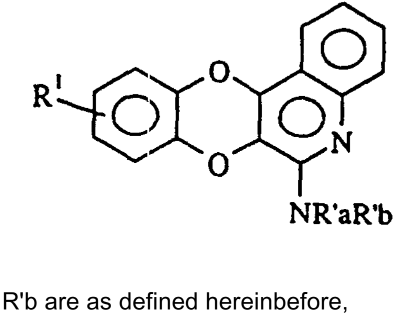

(I/f)

wherein $R^1$, R'a and R'b are as defined hereinbefore,

- **or** with 2-iodotoluene to obtain the compound of formula (XII):

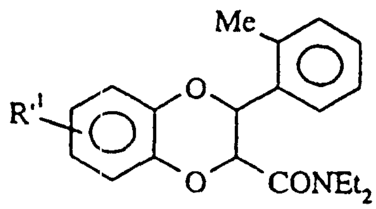

(XII)

wherein $R'^1$ is as defined hereinbefore, which, after the action of a strong base, such as lithium diisopropylamide, yields the compound of formula (I/g), a particular case of the compounds of formula (I):

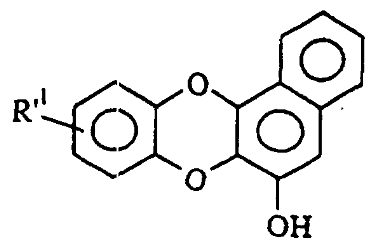

(I/g)

wherein $R'^1$ is as defined hereinbefore,
which compound of formula (I/g) is subjected:

* when $R'^1$ represents a linear or branched $(C_1\text{-}C_6)$alkoxy group, to the action of a dealkylating agent, such as boron tribromide for example, to yield the compound of formula (I/h), a particular case of the compounds of formula (I):

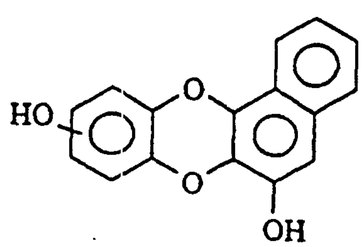

(I/h)

* or to the successive action of trifluoromethanesulphonic anhydride, a cyanide in the presence of a complex of a transition metal such as palladium, and a reducing agent, such as diisobutyl aluminium hydride, to obtain the compound of formula (I/i), a particular case of the compounds of formula (I):

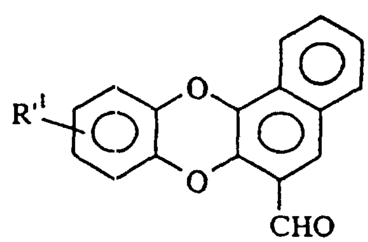

(I/i)

wherein $R'^1$ is as defined hereinbefore, which is oxidised to the methyl ester by the action of an oxidising agent such as manganese oxide in the presence of methanol, to yield the compound of formula (I/j), a particular case of the compounds of formula (I):

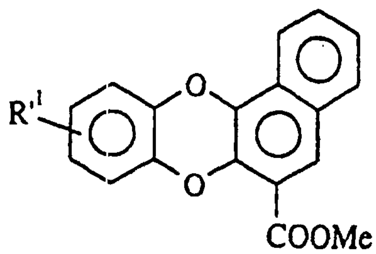

(I/j)

wherein R'[1] is as defined hereinbefore, which, when R'[1] represents a linear or branched ($C_1$-$C_6$)alkoxy group, is reacted with a dealkylating agent, such as boron tribromide, to yield the compound of formula (I/k) a particular case of the compounds of formula (I):

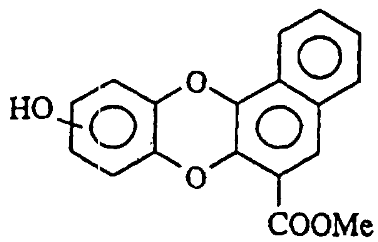

(I/k)

the totality of the compounds (I/j) and (I/k) constituting the compound of formula (I/1), a particular case of the compounds of formula (I):

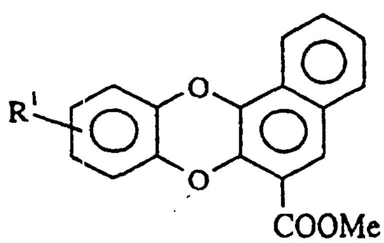

(I/l)

wherein $R^1$ is as defined for formula (I), which is condensed with diamines of formula HNR'aR'b wherein R'a represents a di-($C_1$-$C_6$)alkyl-($C_1$-$C_6$)aminoalkyl group (each alkyl moiety being linear or branched and identical or different) and R'b represents a hydrogen atom or a linear or branched ($C_1$-$C_6$) alkyl group, to yield the compound of formula (I/m), a particular case of the compounds of formula (I):

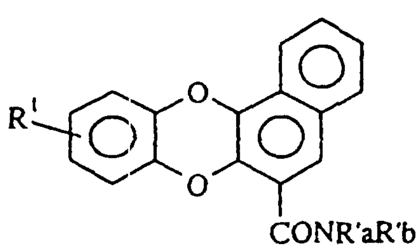

(I/m)

wherein $R^1$, R'a and R'b are as defined hereinbefore, it being understood that the compounds (I/m) can be obtained starting from compound (I/k) which is subjected to electrophiles of formula $R'^1$-halogen wherein $R'^1$ is as defined hereinbefore, in a basic medium,

- *for compounds wherein X does not represent a nitrogen atom,* the compound of formula (XIII):

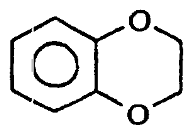

(XIII)

which is:

- either dibrominated and then treated with metachloroperbenzoic acid and subjected to the action of potassium iodide to obtain the compound of formula (XIIIa):

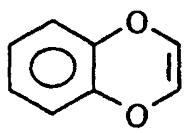

(XIIIa)

- or acylated according to a Friedel Crafts reaction, dibrominated, treated with metachloroperbenzoic acid, subjected to the action of sodium iodide and then of a base to obtain the compound (XIV):

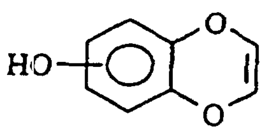

(XIV)

which is condensed with electrophiles of formula R'''-halogen wherein R''' represents a linear or branched ($C_1$-$C_6$)alkyl group, optionally substituted by an aryl or heteroaryl group, to yield the compound of formula (XV):

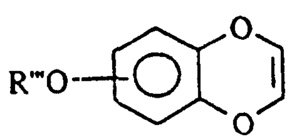

(XV)

wherein R''' is as defined hereinbefore, the totality of the compounds (XIIIa) and (XV) constituting the compound of formula (XVI):

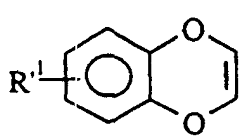

(XVI)

wherein $R'^1$ represents a hydrogen atom, a linear or branched ($C_1$-$C_6$)alkoxy group, optionally substituted by

an aryl or heteroaryl group, which, after the action of a strong base, such as butyllithium, for example, is condensed with electrophiles of formula (XVII):

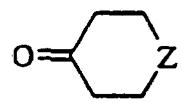

(XVII)

wherein Z represents a group NCOOEt or a group

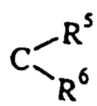

wherein $R^5$ and $R^6$ simultaneously represent a hydrogen atom or together form a ring

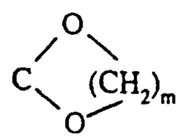

wherein m is 2 or 3,
to yield the compound of formula (XVIII):

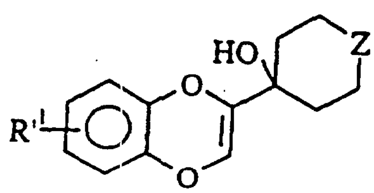

(XVIII)

wherein $R'^1$ and Z are as defined hereinbefore, which is dehydrated in the presence of mesyl chloride for example, to obtain the compound of formula (XIX):

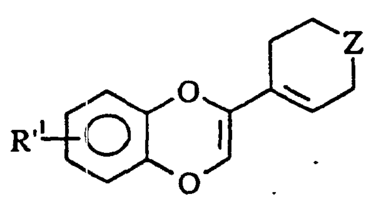

(XIX)

wherein $R'^1$ and Z are as defined hereinbefore, which is condensed with dimethyl acetylenedicarboxylate or methyl propiolate, to obtain the compound of formula (XX):

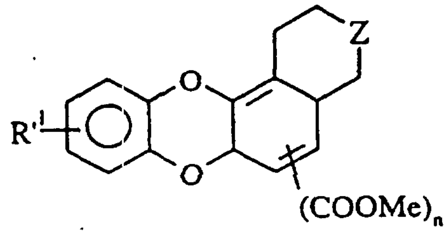

(XX)

wherein $R'^1$ and Z are as defined hereinbefore and n is 1 or 2, which is subjected to the action of an oxidising agent, such as orthochloranil or 2,3-dichloro-5,6-dicyano-1,4-benzoquinone, to yield

- the compound of formula (I/n), a particular case of the compounds of formula (I):

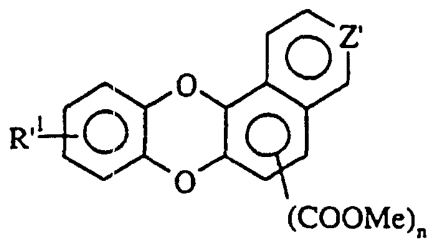

(I/n)

wherein $R'^1$ and n are as defined hereinbefore and Z' represents a nitrogen atom or a C-$R^7$ group wherein $R^7$ represents a hydrogen atom or an O-$(CH_2)_m$-OH group wherein m is 2 or 3,

- or the compound of formula (XXI):

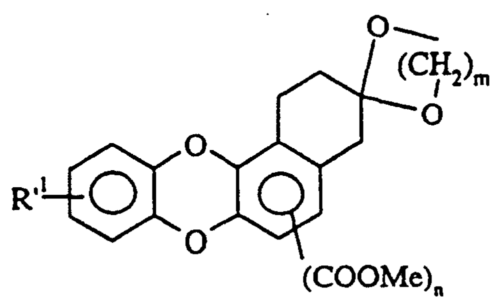

(XXI)

wherein $R'^1$, n and m are as defined hereinbefore, which is successively subjected to deblocking of the acetal in an acidic medium, the formation of a silyl ether, oxidisation with 2,3-dichloro-5,6-dicyano-1,4-benzoquinone, and deblocking of the silyl ether with a fluorinated agent, to yield the compound of formula (I/o), a particular case of the compounds of formula (I):

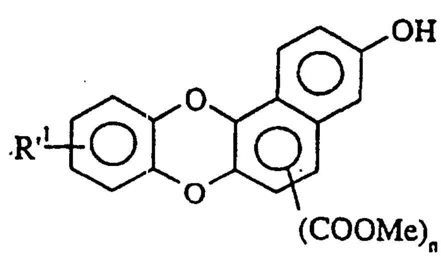

(I/o)

wherein $R'^1$ and n are as defined hereinbefore, which is condensed with various electrophiles $R^8$-halogen

wherein $R^8$ represents a linear or branched $(C_1-C_6)$alkyl group, optionally substituted by an aryl or heteroaryl group, to obtain the compounds of formula (I/p), a particular case of the compounds of formula (I):

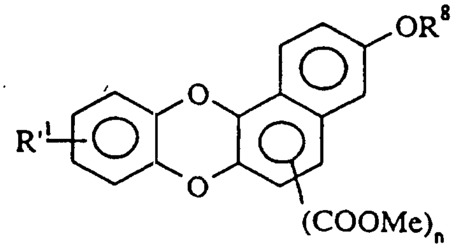
(I/p)

wherein $R'^1$, n and $R^8$ are as defined hereinbefore, which compounds of formulae (I/n), (I/o) and (I/p) constitute the totality of the compounds of formula (I/q), a particular case of the compounds of formula (I):

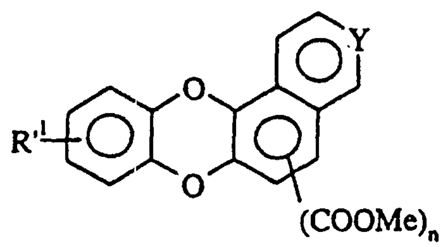
(I/q)

wherein $R'^1$ and n are as defined hereinbefore and Y is as defined for formula (I), which compound (I/q):

* when n is 1 or 2, is condensed with diamines of formula HNR"aR"b wherein R"a represents a di-$(C_1-C_6)$alkyl-$(C_1-C_6)$aminoalkyl group (each alkyl moiety being linear or branched and identical or different independently of the others) or a linear or branched $(C_1-C_6)$alkyl group, substituted by at least one hydroxy group, and R"b represents a hydrogen atom or a linear or branched $(C_1-C_6)$alkyl group, to yield the compounds of formula (I/r), a particular case of the compounds of formula (I):

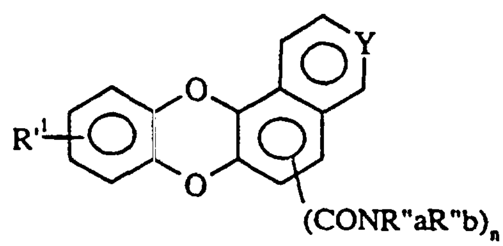
(I/r)

wherein $R'^1$, R"a, R"b, n and Y are as defined hereinbefore,

* or, when n is 1 or 2, is subjected to reductive conditions to yield the corresponding alcohol, which is subjected to oxidation to the aldehyde in the presence of manganese oxide, to yield the compound of formula (XXII):

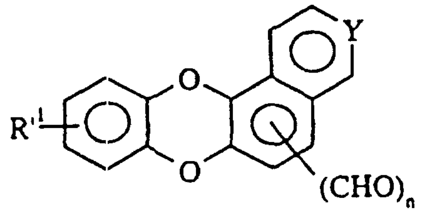

(XXII)

wherein $R'^1$, Y and n are as defined hereinbefore,
which is condensed, in the presence of zinc chloride and sodium cyanoborohydride, with diamines of formula HNR"aR"b wherein R"a represents a di-($C_1$-$C_6$)alkyl-($C_1$-$C_6$)aminoalkyl group in which each alkyl moiety is linear or branched, or a linear or branched ($C_3$-$C_6$)alkyl group substituted by at least one hydroxy group, and R"b represents a hydrogen atom or a linear or branched ($C_1$-$C_6$)alkyl group, to yield the compounds of formula (I/s), a particular case of the compounds of formula (I):

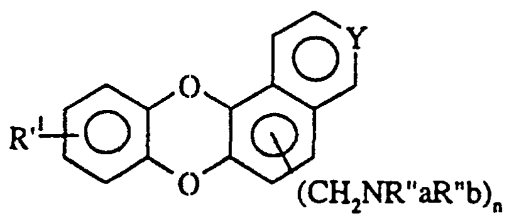

(I/s)

wherein $R'^1$, R"a, R"b, Y and n are as defined hereinbefore,

* or when n is 2, the compounds of formula (I/t), a particular case of the compounds of formula (I):

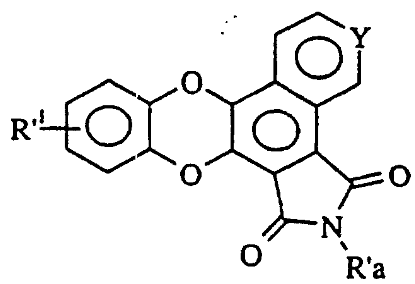

(I/t)

wherein $R'^1$, R'a and Y are as defined hereinbefore,
which compounds (I/a-t) constitute the totality of the compounds of the invention, which are purified, if necessary, according to a conventional purification technique, which may, if desired, be separated into their various optical or geometric isomers or converted into salts with a pharmaceutically acceptable acid or base.

**9.** Pharmaceutical compositions comprising as active ingredient at least one compound according to any one of claims 1 to 7, alone or in combination with one or more inert, non-toxic, pharmaceutically acceptable excipients or carriers.

**10.** Pharmaceutical compositions according to claim 9 comprising at least one active ingredient, an anti-tumour agent according to any one of claims 1 to 7, for use in the treatment of cancers.